# EUROPEAN PATENT APPLICATION

(11) **EP 4 400 603 A2**
(43) Date of publication of application: **17.07.2024**
(21) Application number: 24159811.9
(22) Date of filing: 10.02.2017
(51) Int. Cl.: C12Q 1/6869

(54) **METHOD FOR TARGET SPECIFIC RNA TRANSCRIPTION OF DNA SEQUENCES**

(30) Priority: 12.02.2016 US 201662294875 P
(62) Divisional of application: 17706942.4
(71) Applicant: Jumpcode Genomics, Inc., San Diego, CA 92121 (US)
(72) Inventor: BROWN, Keith, Carlsbad, CA 92008 (US)
(74) Representative: J A Kemp LLP

(57) **Abstract**

Disclosed herein are methods of long range target specific amplification and sequencing using an RNA intermediate synthesized directly from the target to eliminate clonal amplification of early synthesis errors. Approaches allow for the identification of target-adjacent sequence, such as sequence adjacent to a repeat element target. Also disclosed herein are compositions and kits for amplification and sequencing.

## Description

### RELATED APPLICATIONS

The present application claims the benefit of U.S. Provisional Application Serial No. 62/294,875, filed February 12, 2016, the contents of which are hereby incorporated by reference in their entirety.

### BACKGROUND

The disclosure herein relates to the field of molecular biology, such as amplification and identification of nucleic acid sequence adjacent to repetitive sequence in a nucleic acid sample.

PCR, or variants of PCR technology along with hybrid capture are dominant methods of targeted sequencing. Although widely used, both have limitations for long read sequencers. Hybrid capture uses short RNA or DNA probes with biotin to hybridize to target DNA and "pull down" the sequences of interest. For long target sequences, this approach is inefficient, both because many oligonucleotide probes are required and because the process often results in physical shearing of the long DNA molecules during the pulldown process. These defects limit the length of the contiguous sequencer read using single molecule technologies.

Long range PCR has been used as an alternative, but also presents challenges. Long range PCR is hard to multiplex. Often, one loses the ability to detect large chromosomal events such as translocations due to the requirement of opposing PCR primers on opposite strands outside of the target region. In addition, the clonal amplification of PCR limits sensitivity to detect low frequency somatic variation in a heterogeneous sample such as a tumor, and may propagate polymerase errors such as point mutations or translocations from the early cycles of the reaction. Further, long-range PCR sometimes exhibits template switching, creating errors in the amplification product.

### SUMMARY

Advances in genome sequencing technologies have greatly increased our understanding of human genetic variation and its contribution to disease. Short read DNA sequencing technologies (Illumina, Thermo Fisher, Qiagen) produce billions of short reads resulting in the routine identification of single nucleotide polymorphisms and small insertions and deletions. These short read sequencing technologies have not shown a sensitivity to detect more complex variation such as large scale chromosomal rearrangements, translocations and mobile element rearrangements. Long read sequencing technologies (Pacific Biosciences, Oxford Nanopore) have shown the ability to generate single molecule read lengths in excess of 10,000 base pairs, but do not have the capacity to sequence and assemble a full human genome. Targeted strategies disclosed herein take advantage of these longer read lengths.

Here we describe methods of long range target specific amplification where only the original template is amplified to produce increased copies of the original target sequence relative to the sample DNA sequence. Amplified products are derived directly from the sample template rather than from synthesized amplification intermediates or previously synthesized copies of the sample template. As a result synthesized copies do not incorporate errors from a prior synthesis reaction. This dramatically reduces the chance that early errors may be differentially amplified during a reaction. Because synthesis products do not serve as templates, any errors in synthesis are independently derived, and are unlikely to match from one molecule to the next. Accordingly by comparing synthesized products, one can readily identify errors in synthesis and more readily derive the sample sequence.

The disclosed subject matter is summarized in part in the listing of the claims which accompanies this disclosure.

Provided herein are methods of determining a sequence adjacent to a region of known sequence of a nucleic acid molecule. Some such methods comprise a) attaching a nucleic acid fragment comprising promoter sequence at the known region of the nucleic acid molecule; b) contacting the nucleic acid fragment to an RNA polymerase directed by the promoter; and c) synthesizing a plurality of RNA molecules; wherein a consensus sequence of the plurality of RNA molecules represents the sequence adjacent to the known region of a nucleic acid molecule. Optionally, the consensus sequence is at least 10 kilobases in length. Sometimes, the method comprises treating the nucleic acid molecule using a DNase subsequent to synthesizing the plurality of RNA molecules. Alternately or in combination, the method comprises reverse-transcribing the plurality of RNA molecules. The method sometimes comprises determining nucleic acid sequences of the plurality of RNA molecules. Optionally, the consensus sequence of the plurality of RNA molecules comprises sequence of molecules synthesized directly from the nucleic acid molecule. Alternately or in combination, the attaching comprises inserting the nucleic acid fragment comprising promoter sequence at the known region of the nucleic acid molecule. In some cases, the attaching comprises inserting the nucleic acid fragment comprising promoter sequence at the region of known sequence of the nucleic acid molecule. Optionally, the attaching comprises sequence-specific cleavage of the region of known sequence of the nucleic acid molecule. Alternately or in combination, the attaching comprises contacting the known region of the nucleic acid molecule to a CRISPR nucleic acid-protein complex. Optionally, the CRISPR nucleic acid-protein complex comprises a guide RNA comprising SEQ ID NO: 3. In some cases, the attaching comprises ligating the nucleic acid fragment comprising promoter sequence. Sometimes the nucleic acid fragment comprising promoter sequence comprises a viral promoter. Optionally, the viral promoter binds a viral RNA polymerase and is at least one promoter selected from the list consisting of T7, T3, T7lac, SP6, pL, CMV, SV40, and CaMV35S. Alternately or in combination, the nucleic acid fragment comprising promoter sequence comprises a bacterial promoter. In some cases, the bacterial promoter binds a bacterial RNA polymerase and is at least one promoter selected from the list consisting of araBAD, trp, lac, and Ptac. Sometimes, the nucleic acid fragment comprising promoter sequence comprises a eukaryotic promoter. Optionally, the eukaryotic promoter binds a eukaryotic RNA polymerase and is at least one promoter selected from the list consisting of EF1a, PGK1, Ubc, beta actin, CAG, TRE, UAS, Ac5, Polyhedrin, CaMKIIa, ALB, GAL1, GAL10, TEF1, GDS, ADH1, Ubi, H1, and U6. Alternately or in combination, the eukaryotic promoter is at least one promoter selected from the list consisting of an RNA pol I promoter, an RNA pol II promoter and an RNA pol III promoter. Optionally, the known region of a nucleic acid molecule comprises a repetitive element. In some cases, the repetitive element comprises a mobile insertion element. Sometimes, the repetitive element comprises at least one of a LINE element, a SINE element, an Alu repeat, a transposon, a retrotransposon, a centromeric repeat, and a telomeric repeat. Alternately or in combination, the LINE element comprises SEQ ID NO: 1.

In additional embodiments, there are provided methods of determining a plurality of locus-adjacent sequences of an element in a nucleic acid sample, comprising the steps of a) inserting a nucleic acid comprising a promoter into the element, b) generating a plurality of nucleic acid molecules directed by the promoter, and c) determining the sequence of the plurality of nucleic acid molecules, wherein the nucleic acid molecules are synthesized directly from the nucleic acid sample and wherein the plurality of nucleic acid molecules span locus adjacent sequences. Optionally, the nucleic acid molecules comprise RNA. In some cases, the nucleic acid molecules cannot prime nucleic acid synthesis. Sometimes, the nucleic acid sample comprises cancer cell nucleic acids. In some cases, the nucleic acid sample comprises a single nuclear genome. Often, the nucleic acid sample is obtained from a single cell. Optionally, the method comprises treating the nucleic acid sample using a DNase subsequent to synthesizing the plurality of RNA molecules. Sometimes, the method comprises reverse-transcribing the plurality of RNA molecules. In some cases, the plurality of nucleic acid molecules are RNA molecules. Sometimes, the consensus sequence of the plurality of RNA molecules comprises sequence of molecules synthesized directly from the nucleic acid molecule. In some cases, the attaching comprises inserting the nucleic acid fragment comprising promoter sequence at the known region of the nucleic acid molecule. Optionally, the attaching comprises inserting the nucleic acid fragment comprising promoter sequence at the known region of the nucleic acid molecule. Sometimes, the attaching comprises sequence-specific cleavage of the known region of the nucleic acid molecule. Optionally, the attaching comprises contacting the known region of the nucleic acid molecule to a CRISPR nucleic acid-protein complex. In some cases, the CRISPR nucleic acid-protein complex comprises a guide RNA comprising SEQ ID NO: 3. Sometimes, the attaching comprises ligating the nucleic acid fragment comprising promoter sequence. In some cases, the nucleic acid fragment comprising promoter sequence comprises a viral promoter. A viral promoter is variously at least one promoter selected from the list consisting of T7, T3, T7lac, SP6, pL, CMV, SV40, and CaMV35S. Sometimes, the nucleic acid fragment comprising promoter sequence comprises a bacterial promoter. Optionally, the bacterial promoter is at least one promoter selected from the list consisting of araBAD, trp, lac, and Ptac. In some cases, the nucleic acid fragment comprising promoter sequence comprises a eukaryotic promoter. For example, sometimes the eukaryotic promoter is at least one promoter selected from the list consisting of EF1a, PGK1, Ubc, beta actin, CAG, TRE, UAS, Ac5, Polyhedrin, CaMKIIa, ALB, GAL1, GAL10, TEF1, GDS, ADH1, Ubi, H1, and U6. Optionally, the eukaryotic promoter is at least one promoter selected from the list consisting of an RNA pol I promoter, an RNA polII promoter and an RNA polIII promoter. Sometimes, the known region of a nucleic acid molecule comprises a repetitive element. Some repetitive element comprises a mobile insertion element. In some cases, the repetitive element comprises at least one of a LINE element, a SINE element, an Alu repeat, a transposon, a retrotransposon, a centromeric repeat, and a telomeric repeat. Optionally, the LINE element comprises SEQ ID NO: 1.

Also provided herein, in some embodiments, are nucleic acid libraries comprising nucleic acids encoding border adjacent sequence for at least 90% of a repeated mobile element's borders in a nucleic acid sample. Sometimes, discrepancies between library constituents and the nucleic acid sample are independently derived. Optionally, at least 50% of said repeated element's borders are present in at least 100 copies. In some cases, library constituents are derived directly from the nucleic acid sample. Alternately or in combination, library components are not clonally amplified prior to sequencing . Optionally, the nucleic acid sample is derived from a single cell. Sometimes, the nucleic acid library is reverse transcribed from an RNA intermediate. In some cases, the nucleic acid library comprises RNA. Optionally, nucleic acid library constituents comprise promoter sequence. Optionally, the RNA promoter sequence comprises at least one of a T7, T3, T7lac, SP6, pL, CMV, SV40, CaMV35S, araBAD, trp, lac, Ptac, EF1a, PGK1, Ubc, beta actin, CAG, TRE, UAS, Ac5, Polyhedrin, CaMKIIa, ALB, GAL1, GAL10, TEF1, GDS, ADH1, Ubi, H1, and U6. Optionally, at least one border adjacent sequence indicates a defect in a gene related to at least one of cell cycle regulation, DNA repair, and growth regulation. In some cases, the nucleic acid library comprises nucleic acids encoding border adjacent sequence for at least 95% of a repeated mobile element's borders in a nucleic acid sample. In some cases, the nucleic acid library comprises nucleic acids encoding border adjacent sequence for at least 99% of a repeated mobile element's borders in a nucleic acid sample. Alternately or in combination, at least 50% of the library constituent nucleic acids are located on a nucleic acid within 20kb of a mobile element border. In some cases, at least 75% of the library constituent nucleic acids are located on a nucleic acid within 20kb of a mobile element border in proximity to a mobile element border. Optionally, at least 90% of the library constituent nucleic acids are located on a nucleic acid within 20kb of a mobile element border. Sometimes, at least 50% of the library constituent nucleic acids are located on a nucleic acid within 10kb of a mobile element border. In some cases, at least 75% of the library constituent nucleic acids are located on a nucleic acid within 10kb of a mobile element border in proximity to a mobile element border. In some cases, at least 90% of the library constituent nucleic acids are located on a nucleic acid within 10kb of a mobile element border. Optionally, at least 50% of the library constituent nucleic acids are located on a nucleic acid within 5kb of a mobile element border. Sometimes, at least 75% of the library constituent nucleic acids are located on a nucleic acid within 5kb of a mobile element border in proximity to a mobile element border. In some cases, at least 90% of the library constituent nucleic acids are located on a nucleic acid within 5kb of a mobile element border. Optionally, at least 50% of the library constituent nucleic acids are located on a nucleic acid within 1kb of a mobile element border. In some cases, at least 75% of the library constituent nucleic acids are located on a nucleic acid within 1kb of a mobile element border in proximity to a mobile element border. Optionally, at least 90% of the library constituent nucleic acids are located on a nucleic acid within 1kb of a mobile element border. Sometimes, the mean fragment length is about 500 bases. In some cases, the mean fragment length is about 1000 bases. Optionally, the median fragment length is about 500 bases. Optionally, the median fragment length is about 1000 bases.

In further embodiments, there are provided compositions comprising a targeting sequence and a promoter, wherein the targeting sequence comprises a nucleic acid sequence that directs insertion of the composition into one or more specific locations in a nucleic acid sequence and the promoter comprises a nucleic acid sequence that directs synthesis of a nucleic acid from a sample sequence adjacent to the insertion of the promoter. Optionally, the targeting sequence comprises a nucleic acid sequence homologous to the specific location. Sometimes, the targeting sequence comprises a nucleic acid sequence that base pairs to the specific location. In some cases, the targeting sequence comprises a nucleic acid sequence that hybridizes to the specific location. In some cases, the targeting sequence comprises at least one of clustered regularly interspaced short palindromic repeats (CRISPR) sequence, a zinc finger nuclease (ZFN) sequence, and a transcription activator-like effector nucleases (TALENs) sequence. In some cases, the CRISPR sequence comprises a guide RNA with a sequence comprising SEQ ID NO: 3. Often, the promoter comprises at least one of a bacterial promoter, a viral promoter, and a eukaryotic promoter. In some cases, the bacterial promoter comprises at least one of araBAD, trp, lac, and Ptac. Sometimes, the viral promoter comprises at least one of T7, T7lac, SP6, pL, CMV, SV40, and CaMV35S. The eukaryotic promoter often comprises at least one of EF1a, PGK1, Ubc, beta actin, CAG, TRE, UAS, Ac5, Polyhedrin, CaMKIIa, ALB, GAL1, GAL10, TEF1, GDS, ADH1, Ubi, H1, and U6. Optionally, the specific location in the nucleic acid sequence comprises a low-complexity nucleic acid sequence. Often, the specific location in the nucleic acid sequence comprises a repetitive nucleic acid sequence. Optionally, the low-complexity nucleic acid sequence or the repetitive nucleic acid sequence comprises at least one of a tri-nucleotide repeat, tandem repeat, and human leukocyte antigen gene. In some cases, the specific location in the nucleic acid sequence comprises a mobile genetic element. Optionally, the mobile genetic element comprises at least one of a transposon, a retrotransposon, a DNA transposon, an insertion sequence, a plasmid, a bacteriophage, a group II intron, a group I intron, an Alu element, a MIR element, an intracisternal A particle (IAP), an ETn, a virus, and a fragments thereof. The retrotransposon often comprises at least one of a transposable element, a LINE, a SINE, and fragments thereof. Optionally, the LINE comprises SEQ ID NO: 1. Alternately or in combination, the virus comprises a retrovirus or a fragment thereof. Sometimes, the nucleic acid synthesis comprises at least one of RNA transcription and DNA synthesis.

Also provided herein are methods of determining a nucleic acid sequence adjacent to a nucleic acid sequence of interest comprising: (a) inserting a targeting nucleic acid sequence comprising a targeting sequence and a promoter into one or more specific locations in the nucleic acid sequence of interest, (b) directing synthesis of a nucleic acid from the promoter, and (c) sequencing the synthesized nucleic acid. Some targeting sequences comprise at least one of a clustered regularly interspaced short palindromic repeats (CRISPR) sequence, a zinc finger nuclease (ZFN) sequence, and a transcription activator-like effector nucleases (TALENs) sequence. Optionally, the CRISPR sequence comprises a guide RNA with a sequence comprising SEQ ID NO: 3. In some cases, the promoter comprises at least one of a bacterial promoter, a viral promoter, and a eukaryotic promoter. Optionally, the bacterial promoter comprises at least one of araBAD, trp, lac, and Ptac. Optionally, the viral promoter comprises at least one of T7, T7lac, SP6, pL, CMV, SV40, and CaMV35S. Optionally, the eukaryotic promoter comprises at least one of EF1a, PGK1, Ubc, beta actin, CAG, TRE, UAS, Ac5, Polyhedrin, CaMKIIa, GAL1, GAL10, TEF1, GDS, ADH1, Ubi, H1, and U6. In some cases, the sequence of interest comprises a low-complexity nucleic acid sequence. The sequence of interest often comprises a repetitive nucleic acid sequence. Optionally, the sequence of interest comprises at least one of tri-nucleotide repeat, tandem repeat, and human leukocyte antigen gene. Alternately or in combination, the sequence of interest comprises a mobile genetic element. Optionally, the mobile genetic element comprises a transposon, a retrotransposon, a DNA transposon, an insertion sequence, a plasmid, a bacteriophage, a group II intron, a group I intron, an Alu element, a MIR element, an intracisternal A particle (IAP), an ETn, a virus, or a fragment thereof. Sometimes, the retrotransposon comprises at least one of transposable element, a LINE, a SINE, and fragments thereof. Optionally, the LINE comprises SEQ ID NO: 1. The virus often comprises at least one of a retrovirus and fragments thereof. Optionally, nucleic acid synthesis comprises at least one of RNA transcription and DNA synthesis. In some cases, RNA transcription comprises use of a RNA polymerase. Optionally, the RNA polymerase comprises at least one of a T7 RNA polymerase, a T3 RNA polymerase, a SP6 RNA polymerase, a RNA polymerase I, a RNA polymerase II, a RNA polymerase III, a RNA polymerase IV, a RNA polymerase V, and a single subunit RNA polymerase. In some cases, DNA synthesis comprises use of a DNA polymerase. Optionally, the DNA polymerase comprises at least one of a T7 DNA polymerase, a T3 DNA polymerase, a SP6 DNA polymerase, a DNA polymerase I, a DNA polymerase II, a DNA polymerase III, a Taq DNA polymerase, and a Pfu DNA polymerase. Optionally, the nucleic acid synthesis requires a primer. Often, the synthesized nucleic acid is synthesized directly from the nucleic acid sequence of interest. In some cases, the nucleic acid is synthesized without introducing a mutation. Optionally, the mutation is at least one of a point mutation, a deletion, an insertion, and a chimera. Optionally, the synthesized nucleic acid comprises DNA. In some cases, the synthesized nucleic acid comprises cDNA. Optionally, the synthesized nucleic acid is treated with an RNase. Sometimes, the synthesized nucleic acid is a RNA. Optionally, the synthesized nucleic acid is treated with a DNase. In some cases, the sequencing comprises at least one of Sanger sequencing, Next-generation sequencing, pyrosequencing, Massively parallel signature sequencing, single molecule real-time sequencing, ion torrent sequencing, sequencing by synthesis, and sequencing by ligation. In some cases, the method detects a mutation in a subject. Optionally, the method detects a mutation in a tissue sample obtained from a subject. The tissue sample often comprises at least one of tumor, blood, saliva, sputum, skin, and epithelial tissue.

In additional embodiments provided herein are methods of mapping a site of insertion of a DNA element in a nucleic acid sample from a subject, comprising: i) inserting a targeting nucleic acid sequence comprising a targeting sequence and a promoter by contacting the genomic DNA with the targeting sequence and one or more reagents sufficient to insert the targeting sequence into the DNA element; ii) creating an amplified nucleic acid directly from the genomic DNA by contacting the inserted targeting sequence with one or more enzymes that catalyze nucleic acid synthesis from the promoter thereby creating an amplified nucleic acid; iii) sequencing the amplified nucleic acid. Optionally, the targeting sequence comprises at least one of a clustered regularly interspaced short palindromic repeats (CRISPR) sequence, a zinc finger nuclease (ZFN) sequence, and a transcription activator-like effector nucleases (TALENs) sequence. Sometimes, the CRISPR sequence comprises a guide RNA with a sequence comprising SEQ ID NO: 3. Optionally, the promoter comprises at least one of a bacterial promoter, a viral promoter, and a eukaryotic promoter. In some cases, the bacterial promoter comprises at least one of araBAD, trp, lac, and Ptac. Alternately or in combination, the viral promoter comprises at least one of T7, T7lac, SP6, pL, CMV, SV40, and CaMV35S. Optionally, the eukaryotic promoter comprises at least one of EF1a, PGK1, Ubc, beta actin, CAG, TRE, UAS, Ac5, Polyhedrin, CaMKIIa, GAL1, GAL10, TEF1, GDS, ADH1, Ubi, H1, and U6. Sometimes, the DNA element comprises a low-complexity nucleic acid sequence. In some cases, the DNA element comprises a repetitive nucleic acid sequence. In some cases, the DNA element comprises at least one of a tri-nucleotide repeat, and tandem repeat. Optionally, the DNA element comprises a mobile genetic element. Optionally, the mobile genetic element comprises at least one of a transposon, a retrotransposon, a DNA transposon, an insertion sequence, a plasmid, a bacteriophage, a group II intron, a group I intron, an Alu element, a MIR element, an intracisternal A particle (IAP), an ETn, a virus, and fragments thereof. Optionally, the retrotransposon comprises at least one of a transposable element, a LINE, a SINE, and fragments thereof. Optionally, the LINE comprises SEQ ID NO: 1. Optionally, the virus comprises a retrovirus or a fragment thereof. Sometimes, the enzyme comprises a RNA polymerase. In some cases, the RNA polymerase comprises at least one of a T7 RNA polymerase, a T3 RNA polymerase, a SP6 RNA polymerase, a RNA polymerase I, a RNA polymerase II, a RNA polymerase III, a RNA polymerase IV, a RNA polymerase V, and a single subunit RNA polymerase. Optionally, the enzyme comprises a DNA polymerase. Alternately or in combination, the DNA polymerase comprises at least one of a T7 DNA polymerase, a T3 DNA polymerase, a SP6 DNA polymerase, a DNA polymerase I, a DNA polymerase II, a DNA polymerase III, a Taq DNA polymerase, and a Pfu DNA polymerase. Sometimes, the nucleic acid synthesis requires a primer. Optionally, the synthesized nucleic acid is synthesized directly from the nucleic acid sequence of interest. In some cases, the nucleic acid is synthesized without introducing a mutation. In some cases, is at least one of a point mutation, a deletion, an insertion, and a chimera. Optionally, the synthesized nucleic acid is a DNA. Alternately, the synthesized nucleic acid is a cDNA. Optionally, the synthesized nucleic acid is treated with an RNase. Optionally, the synthesized nucleic acid is a RNA. In some cases, the synthesized nucleic acid is treated with a DNase. Optionally, the sequencing comprises at least one of Sanger sequencing, Next-generation sequencing, pyrosequencing, Massively parallel signature sequencing, single molecule real-time sequencing, ion torrent sequencing, sequencing by synthesis, and sequencing by ligation. Optionally, the method detects a mutation in a subject. Alternately or in combination, the method detects a mutation in a tissue sample obtained from a subject. Optionally, the tissue sample comprises at least one of tumor, blood, saliva, sputum, skin, and epithelial tissue.

Further provided herein are methods of sequencing a repetitive genomic region comprising: i) inserting a targeting nucleic acid sequence comprising a targeting sequence and a promoter by contacting the genomic DNA with the targeting sequence and one or more reagents sufficient to insert the targeting sequence into the repetitive genomic region; ii) creating an amplified nucleic acid directly from the genomic DNA by contacting the inserted targeting sequence with one or more enzymes that catalyze nucleic acid synthesis from the promoter thereby creating an amplified nucleic acid; iii) sequencing the amplified nucleic acid. Optionally, the targeting sequence comprises at least one of a clustered regularly interspaced short palindromic repeats (CRISPR) sequence, a zinc finger nuclease (ZFN) sequence, and a transcription activator-like effector nucleases (TALENs) sequence. Optionally, the CRISPR sequence comprises a guide RNA with a sequence comprising SEQ ID NO: 3. Optionally, the promoter comprises at least one of a bacterial promoter, a viral promoter, and a eukaryotic promoter. Often, the bacterial promoter comprises at least one of araBAD, trp, lac, and Ptac. Optionally, the viral promoter comprises at least one of T7, T7lac, SP6, pL, CMV, SV40, and CaMV35S. The eukaryotic promoter comprises at least one of EF1a, PGK1, Ubc, beta actin, CAG, TRE, UAS, Ac5, Polyhedrin, CaMKIIa, GAL1, GAL10, TEF1, GDS, ADH1, Ubi, H1, and U6. In some cases, the repetitive genomic region comprises a low-complexity nucleic acid sequence. Optionally, the repetitive genomic region comprises a repetitive nucleic acid sequence. In some cases, the repetitive genomic region comprises at least one of a tri-nucleotide repeat and tandem repeat. Sometimes, the repetitive genomic region comprises a mobile genetic element. Optionally, the mobile genetic element comprises at least one of a transposon, a retrotransposon, a DNA transposon, an insertion sequence, a plasmid, a bacteriophage, a group II intron, a group I intron, an Alu element, a MIR element, an intracisternal A particle (IAP), an ETn, a virus, and fragments thereof. In some cases, the retrotransposon comprises at least one of a transposable element, a LINE, a SINE, and fragments thereof. Optionally, the LINE comprises SEQ ID NO: 1. Optionally, the virus comprises a retrovirus or a fragment thereof. Optionally, the enzyme comprises a RNA polymerase. Optionally, the RNA polymerase comprises at least one of a T7 RNA polymerase, a T3 RNA polymerase, a SP6 RNA polymerase, a RNA polymerase I, a RNA polymerase II, a RNA polymerase III, a RNA polymerase IV, a RNA polymerase V, and a single subunit RNA polymerase. Optionally, the enzyme comprises a DNA polymerase. Optionally, the DNA polymerase comprises at least one of a T7 DNA polymerase, a T3 DNA polymerase, a SP6 DNA polymerase, a DNA polymerase I, a DNA polymerase II, a DNA polymerase III, a Taq DNA polymerase, and a Pfu DNA polymerase. In some cases, the nucleic acid synthesis requires a primer. The synthesized nucleic acid is often synthesized directly from the nucleic acid sequence of interest. In some cases, the nucleic acid is synthesized without introducing a mutation. In some cases, the mutation is at least one of a point mutation, a deletion, an insertion, and a chimera. Optionally, the synthesized nucleic acid is a DNA, such as genomic or cDNA. In some cases, the synthesized nucleic acid is treated with an RNase. Optionally, the synthesized nucleic acid is a RNA. In some cases, the synthesized nucleic acid is treated with a DNase. Optionally, the sequencing comprises at least one of Sanger sequencing, Next-generation sequencing, pyrosequencing, Massively parallel signature sequencing, single molecule real-time sequencing, ion torrent sequencing, sequencing by synthesis, and sequencing by ligation. Optionally, the method detects a mutation in a subject. Alternately or in combination, the method detects a mutation in a tissue sample obtained from a subject. Optionally, the tissue sample comprises at least one of tumor, blood, saliva, sputum, skin, and epithelial tissue.

### BRIEF DESCRIPTION OF THE DRAWINGS

Reference is made to the following detailed description that sets forth illustrative embodiments, in which the principles of the invention are utilized, and the accompanying drawings of which:
**Figure 1** shows an exemplary human LINE1 (L1.4) repetitive element DNA sequence (SEQ ID NO: 1).
**Figure 2** shows an exemplary consensus sequence of L1HA: Diagnostic sequence at 3' end of LINE-1 element (SEQ ID NO: 2).
**Figure 3** shows an exemplary guide RNA sequence (SEQ ID NO: 3).
**Figure 4** shows an exemplary Alu-Y sequence (SEQ ID NO:4).
**Figure 5** shows CRISPR induced insertion of target specific T7 promoter sequences.
**Figure 6** shows in vitro transcription of target DNA generating amplified RNA copies of the target template.
**Figure 7** shows T7 promoters specifically inserted upstream and downstream of target sequence..
**Figure 8** shows alternative donor DNA constructs.
**Figure 9** shows results of linear amplification of genomic sequence adjacent to an inserted T7 RNA polymerase site in a sample.

### DETAILED DESCRIPTION

Methods disclosed herein involve nucleic acid editing technology (CRISPR, TALENS, Zinc figure, transposase, and other methods known by one of skill in the art) to insert a bacterial or bacteriophage promoter (such as T7, T3, or SP6, though a wide range of promoters are compatible with the disclosure herein and the list herein is not intended to be exhaustive) capable of DNA directed RNA transcription. Following transcription of RNA molecules from the inserted promoter, there is an effective linear amplification of the adjacent sequence, in the form of a population of RNA molecules derived directly from the sample as template. The RNA molecules are converted by any number of methods into either short read or long read DNA sequencing libraries.

Using CRISPR as an illustrative embodiment, the methods comprise a first design of a sequence specific guide RNA molecule that targets a conserved and locally unique sequence upstream of a sequence of interest. Preferably, the specific guide RNA molecule binds to a nucleic acid sequence that is unique to a repeat element, and may occur multiple times in the sample such as a genomic sample but in which each occurrence corresponds to an occurrence of the repeat element. Optionally, the specific guide RNA molecule binds to a nucleic acid sequence that is unique to a specific genomic region to be sequenced. The sequence specific molecule is added to a mixture of high molecular weight sample DNA, the CRISPR/CAS system components (when using CRISPR technology) and a donor DNA molecule containing the T7 promoter sequence. In some cases the CRISPR/CAS molecule is assembled with its associated nucleic acids prior to contacting to the sample.

After insertion of the RNA promoter such as T7 promoter by CAS into the specific genomic locus, a compatible RNA polymerase such as T7 polymerase is added to the sample along with the requisite ribonucleotides and buffers. In vitro transcription is used to generate an approximate 1,000-fold amplification of target DNA sequences through the RNA intermediate. RNA generated from the in vitro transcription is then used as a template for DNA library generation, such as by fragmentation by synthesis, and conversion into short read sequence libraries. Alternatively, the RNA generated from the in vitro transcription reaction is poly-A tailed or polyadenylated and subsequently reverse transcribed using an oligo dT primer and reverse transcriptase to generate full length reverse transcribed DNA copies of the RNA templates. Adapters are optionally ligated on at this step for subsequent sequencing of full length reverse transcribed DNA molecules using single molecule sequencing technology.

The purpose of some of the methods described herein is to generate long targeted templates suitable for long read sequencing. High molecular weight sample DNA, such as genomic DNA, is first obtained from a source (blood, serum, cells, cell culture, saliva, tumor, hair, skin, epithelial tissue, urine, stool, amniotic fluid, sputum, cerebrospinal fluid, mucus, for example). Standard DNA purification techniques are optionally used for the isolation of high molecular weight sample DNA. The subsequent reactions often occur outside of a cell, though in some embodiments, cellular material may remain in the reaction chamber. Intact cells are not used in the reaction in many embodiments compatible. Alternatively, the targeted template is in the genome of an intact cell. Sample DNA with the methods herein is obtained from any organism. Alternatively, sample DNA is synthetic. Methods for preparing high molecular weight sample DNA are routine and known in the art. In some cases, the sample DNA comprises genomic DNA. Sample DNA comprising genomic DNA is optionally selected from a eukaryotic genome, a prokaryotic genome, a eubacterial genome, an archaea genome, a viral genome, or a synthetic nucleic acid source. In some cases, the sample is a tumor cell or a circulating cancer cell. Alternatively, the sample DNA comprises cell free DNA, plasmid DNA, viral DNA, synthetic DNA, or other high molecular weight DNA samples obtained from a subject.

A guide RNA is designed with a target specific motif in some approaches. The target may be adjacent to or within a gene of interest, adjacent to or within a promoter of interest or within a gene, exon, intron or intergenic region. Guide RNAs use sequences having reverse complementarity to a sample within their sequence to bind to a sample DNA may be complete or incomplete reverse complementarity. Guide RNA may be designed to target multiple positions within the target sequence or flanking the target sequence with insertion of a nucleic acid encoding a donor sequence at any one of either ends of the target sequence and in either or both orientations for insertion of a donor sequence. Guide RNA design is upstream of a photospacer adjacent motif or PAM sequence comprising of NGG nucleotide sequence. Some CAS9 mutants eliminate the need for PAM sequence flanking the target sequence of the guide RNA molecule, and in some cases herein the PAM sequence is absent. Single guide RNAs are used. Alternately, multiple guide RNAs are designed and used simultaneously in a single reaction. In exemplary embodiments guide RNA target sequences are designed at intervals across a target sequence. Guide RNAs are designed to be in opposite orientations along the double stranded target DNA molecule. Optionally guide RNAs are designed to be on opposing strands of the double stranded target DNA molecule. The method may insert a multitude of T7 donor sequences into the target genomic DNA loci of interest.

The donor sequence comprises an amplification site, in some cases specifically a promoter site for T7 polymerase. T3 polymerase and SP6 promoter sequences are alternative donor sequences, as are others available to one of skill in the art. Alternatively, other DNA dependent RNA polymerase promoters are used. In an exemplary embodiment, a T7 promoter is used. A suitable T7 promoter sequence is 5'-TAATACGACTCACTATAG-3' (SEQ ID NO: 5) and T7 transcription starts from the 3' G. RNA transcription occurs 5'-3' generating an RNA molecule also in the 5'-3' orientation, making it a useful template for reverse transcription and conversion into cDNA. RNA polymerases have an extremely low error rate at 0.5 × 10^-4 or one misincorporation for every 10-30kb transcript. RNA copies are generated only from the double stranded template DNA molecule. RNA polymerase fragment lengths range from a few hundred base pairs to multiple kilobases and reported for transcript lengths up to 30 kilobases (T7 ribomax, Promega). Magnesium is needed in the buffer for RNA polymerase amplification.

Incubation times vary according to the promoter and polymerase used. Using T7 polymerase, incubation times range from a few minutes to 2 hours. Longer incubation times typically result in better yields and overall performance. RNA resulting from the in vitro transcription reaction is purified or separated from the DNA sample optionally through DNA degradation using a DNAse or an endonuclease.

Alternatively, the DNA sample is left undegraded. Optionally, RNA fragments are size selected. Downstream processing of RNA templates generated from the in vitro transcription reaction are optionally fragmented, for example though gentle magnesium treatment, physical or enzymatic means. Short RNA fragments are optionally converted to sequencer libraries through standard small RNA library preparation techniques. Alternatively, long RNA molecules are poly-adenylated through poly-A polymerase. Polyadenylated long RNA molecules are reverse transcribed through standard techniques known in the art. Reverse transcriptase enzymes such as SuperScript^{™}, in combination with oligo (d)T primers, are capable of efficiently generating full length cDNA from long polyA tailed RNA molecules. RNA are optionally removed from the reverse transcribed DNA output through digestion of RNA. Single stranded full length reverse transcribed DNA molecules may then be converted to long read sequencer libraries through standard adapter ligation.

Variations at multiple steps in the reaction consistent with the disclosure herein are contemplated. In some variants, donor DNA molecules in the CRISPR/CAS reaction optionally include (in addition to the T7 promoter sequence) a primer site for second strand cDNA synthesis. In these embodiments, the guide RNA plus CRISPR/CAS system makes a double stranded cut at the target site upstream of the PAM sequence. The donor DNA molecule then includes a T7 promoter with a universal primer site downstream. For example, in some instances, the universal primer sequence is a sequencing primer compliment for the sequencer platform of choice. The construct of the donor molecule in the CRISPR/CAS reaction is or comprises 5'-T7 promoter-universal primer sequence-3'. In vitro transcription with T7 polymerase starts at the 3' G of the T7 promoter sequence and transcribe the universal primer sequence downstream and through the target DNA sequence. After polyA addition, oligo (d)T primed reverse transcription will transcribe through the universal primer sequence when creating the first strand cDNA. Second strand cDNA synthesis is achieved by primer extension using reverse compliment primers for the universal primer sequence. The final construct of the cDNA molecule entering NGS library conversion would therefore be 5'-universal primer-target DNA-polyA-3'.

In alternative methods contemplated herein, the CRISPR/CAS system makes a double stranded cut at the target site. The double stranded cut is treated with an exonuclease which creates a sticky end of single stranded DNA at the cut site to which an adapter having complementary sequence and a transcription promoter anneals. The annealed adaptor is ligated to the sample DNA, creating a promoter-target DNA hybrid ready for in vitro transcription of the sample DNA. In this alternative method, two levels of specificity are introduced both at the CRISPR/CAS sequence specific targeted double stranded DNA cleavage and at the annealing of the adaptor at the exonuclease created sticky end. Optionally, the adaptor is a hairpin comprising a portion which folds onto itself and a second portion which has a sequence complementary to a targeted sequence. Hairpin adaptors may have advantages such as improved ligation efficiency and kinetics.

Addition of a molecular barcode upstream of the T7 promoter sequence (and the optional universal primer sequence) in the donor DNA molecule is employed in some cases. The construct of the donor DNA sequence in this example is 5'-T7 promoter-universal primer-N-mer-3'. In some cases, PCR amplification is required to increase the amount of material for sequencer library generation and the addition of the molecular barcode enables the identification of unique molecules from clonally amplified long PCR products. For single cell applications using massively parallel compartmentalization through microdroplet or microfluidic technologies, the molecular barcode is optionally replaced by a compartment specific barcode. This allows for bulk processing of all cells in the sample post CRISPR/CAS target insertion and allows for the unique identification of sequenced molecules from a given single cell.

Approaches presented herein often allow the targeted amplification of a plurality of repeat-adjacent nucleic acid sequences. Thus, one is enabled to determine, for example, the global distribution of insertion sites throughout a nucleic acid sample such as a genome. In particular, by selecting a genomic mobile element, one determines a plurality or regions where the mobile element is inserted, up to and including 10%, 20%, 30%, 40%, 50%, 60%, 70%, 80%, 90%, 95%, 99%, or even 100% of the total number of insertion sites for a repetitive element of interest in a genome. In some cases a portion of these insertion sites are amplified 10x, 100x, 1000x, 10000x, 100000x, 1000000x or greater. This fraction amplified to this level comprises up to and including 10%, 20%, 30%, 40%, 50%, 60%, 70%, 80%, 90%, 95%, 99%, or even 100% of the total number of insertion sites in some cases.

Thus, through practice of the methods and use of the compositions disclosed herein, one is able to determine the global insertion pattern for a mobile repeat element throughout the entire genome of a cell or cell population of interest, such as a cancer cell or cancer cell population. Furthermore, through the high level of amplification achieved, one is able to study single cells or very small cell populations. Finally, because the amplification is in many cases linear amplification directly generated from the sample template, rather than a product of exponential amplification using PCR the amplification is accomplished without any concomitant proliferation of errors such as point mutations or translocations that occur pursuant to the library generation process. Errors may occur, but are easily recognized as they are very likely to be unique to a single product. Furthermore, because the amplification occurs through an RNA intermediate, there is no risk of a synthesized product annealing to the sample or another amplified product and priming further extension, a phenomenon which is difficult to distinguish from a translocation event in the sample itself.

Methods and use of the compositions disclosed herein allow one to determine the sequence at any targeted site in the genome, including repetitive elements as well as average complexity DNA sequences, for example mRNA coding sequences. Accordingly, methods herein are not limited to sequencing repetitive and low-complexity genomic regions but can be applied to any desired location in the genome.

As a result of practice of methods disclosed herein, one obtains a library that is both highly amplified, highly representative of the total distribution sites for a mobile element, and highly resistant to error propagation in the synthesis process.

Methods, compositions and kits are provided for producing multi-insert nucleic acids. These methods, compositions and kits find use in a number of applications, such as whole-genome sequencing. These and other objects, advantages, and features of the invention will become apparent to those persons skilled in the art upon reading the details of the compositions and methods as more fully described below.

### Targeted insertion of Tags

Disclosed herein are methods of obtaining sequences from a desired genomic location by inserting a tag into the desired genomic location. In some cases, tags are inserted into the desired genomic location using gene targeting technology, for example CRISPR, TALENS, Zinc figure, transposase, and other methods known by one of skill in the art. The tag is designed to contain a sequence that allows amplification from the desired genomic location. The tag is chosen depending on the desired method of amplification. In some cases, the desired genomic location is amplified by transcription. If the desired genomic location is amplified by transcription, the tag is designed to contain a promoter sequence, for example a bacteriophage promoter such as T7, T3, SP6, or other bacteriophage promoter. The promoter sequence, in some cases, is a viral promoter such as pL, CMV, SV40, CaMV35S, or other viral promoter. In some cases, it is desirable to use a mammalian promoter sequence such as EF1a, PGK1, Ubc, beta actin, CAG, TRE, UAS, Ac5, Polyhedrin, CaMKIIa, ALB, GAL1, GAL10, TEF1, GDS, ADH1, Ubi, H1, U6, or other mammalian promoter. In some cases, the promoter is a RNA polymerase I promoter. In some cases, the promoter is a RNA polymerase II promoter. In some cases, the promoter is a RNA polymerase III promoter. In some cases, the promoter is a RNA polymerase IV promoter. In some cases the promoter is a RNA polymerase V promoter. In some cases, the promoter is a single subunit RNA polymerase promoter.

Tags comprising CRISPR targeting sequences include but are not limited to the group consisting of ACTAGAAAATCTAGAAGAAA (SEQ ID NO: 5), TTGTAGTATAGTTTGAAGTC (SEQ ID NO: 6), AAAACCCTAGAAGAAAACCT (SEQ ID NO: 7), TCTTTAAGAATGTTGAATAT (SEQ ID NO: 8), ACAGCCAATATCATACTGAA (SEQ ID NO: 9), TCACATAGTCCCATATTTCT (SEQ ID NO: 10), CTACAGTAACCAAAACAGCA (SEQ ID NO: 11), AGCAACTTCAGCAAAGTCTC (SEQ ID NO: 12), TGACTTCAAACTATACTACA (SEQ ID NO: 13), TAAGCTTTTTGATGTGCTGC (SEQ ID NO: 14), CCTCCCTAACTCATTTTATG (SEQ ID NO: 15), GAAGCATTCCCTTTGAAAAC (SEQ ID NO: 16), ACCTGCTCCTGAATGACTAC (SEQ ID NO: 17), TGAAGTTGCTTATCAGCTTA (SEQ ID NO: 18), GAGTTCTGTAGATGTCTATT (SEQ ID NO: 19), TATTCACAATAGCAAAGACT (SEQ ID NO: 20), TTGTCTCTTTTGATCTTTGT (SEQ ID NO: 21), TTGAACCAGCCTTGCATCCC (SEQ ID NO: 22), AGGATTCCCTATTTAATAAA (SEQ ID NO: 23), TTGCCCATTCAGTATGATAT (SEQ ID NO: 24), GTTCTTTTAATTGTGATGTT (SEQ ID NO: 25), AAGATCAAAAGAGACAAAGA (SEQ ID NO: 26), TTCACTTATGAAGCTTAGTT (SEQ ID NO: 27), AAACTAAGCTTCATAAGTGA (SEQ ID NO: 28), AAAAATCCTCAATAAAATAC (SEQ ID NO: 29), CATCTATTGAGATAATCATG (SEQ ID NO: 30), CCCAGCACCATTTATTAAAT (SEQ ID NO: 31), TCCTGAATACAGCACACTGA (SEQ ID NO: 32), TGTCTTGTGCCAGTTTTCAA (SEQ ID NO: 33), TTTGATTTGCATTTCTCTGA (SEQ ID NO: 34), ATCCCTTTACCATTATGTAA (SEQ ID NO: 35), TGAGAGATTTTGTCACCACC (SEQ ID NO: 36), AATCTGACAATTATGTGTCT (SEQ ID NO: 37), CAGTTTCAGCTTTCTACATA (SEQ ID NO: 38), CATATGTAGAAAGCTGAAAC (SEQ ID NO: 39), AATATATATGCACCCAATAC (SEQ ID NO: 40), GATGGTAGTTTGTATTTCTG (SEQ ID NO: 41), AGTCTGTTTTATCAGAGACT (SEQ ID NO: 42), GCCAGTCTGTGTCTTTTAAT (SEQ ID NO: 43), CTTCCAACACTATGTTGAAT (SEQ ID NO: 44), AAACTACTTTAAAGTTCATA (SEQ ID NO: 45), AATGTGGCACATATACACCA (SEQ ID NO: 46), CACATTCAAAAGCTAGCAGA (SEQ ID NO: 47), CCCATCAGTGTGCTGTATTC (SEQ ID NO: 48), ATCTTTCCTGCTTTCTCTTG (SEQ ID NO: 49), CTAAGCCAAAAGAACAAAGC (SEQ ID NO: 50), TCATCCCTGGGATGCAAGGC (SEQ ID NO: 51), CTCTTTGAAGCAATTGTGAA (SEQ ID NO: 52), GCCCATGCCTATGTCCTGAA (SEQ ID NO: 53), TGCCTCCAGCTTTGTTCTTT (SEQ ID NO: 54), TTTTTCCTTCATTTCAACTT (SEQ ID NO: 55), AAACTACCATCAGAGTGAAC (SEQ ID NO: 56), AGGAAAACTAACAAACAGAA (SEQ ID NO: 57), TCAAAGAGAATAAAATACCT (SEQ ID NO: 58), AAATGCCCACAAGAGAAAGC (SEQ ID NO: 59), AATGACTTTCTTCACAGAAT (SEQ ID NO: 60), ATTCACCAAAGTTGAAATGA (SEQ ID NO: 61), AATTCTGTGAAGAAAGTCAT (SEQ ID NO: 62), ATTCCAATCAATAGAAAAAG (SEQ ID NO: 63), CCTGTCATTATGATGTTAGC (SEQ ID NO: 64), CCAGCTAACATCATAATGAC (SEQ ID NO: 65), TGACCCAGCCATCCCATTAC (SEQ ID NO: 66), TACCATTCAGGACATAGGCA (SEQ ID NO: 67), CTGTTCTTTTACATTTGCTG (SEQ ID NO: 68), GATCTGTCTAATGTTGACAG (SEQ ID NO: 69), GTTCTAGTTTGATTGCACTG (SEQ ID NO: 70), TTCCCTCTTTTTCTATTGAT (SEQ ID NO: 71), TTAAAAAGTCAGGAAACAAC (SEQ ID NO: 72), ACACAACATACCAGAATCTC (SEQ ID NO: 73), AGGAAGATCTACCAAGCAAA (SEQ ID NO: 74), GTAAACTAGTTCAACCATTG (SEQ ID NO: 75), GTGCAATCAAACTAGAACTC (SEQ ID NO: 76), ACTCCTATTCAACATAGTGT (SEQ ID NO: 77), GCAGAGCTGAGTTCAATTCC (SEQ ID NO: 78), CCATCTCACACCAGTTAGAA (SEQ ID NO: 79), CCTTCACATCCCTTGTAAGT (SEQ ID NO: 80), TATCTCAATAGATGCAGAAA (SEQ ID NO: 81), TTAAGGGCAGCCAGAGAGAA (SEQ ID NO: 82), CTAAAAACTCTCAATAAATT (SEQ ID NO: 83), TATGTACCCAGTAGTCATTC (SEQ ID NO: 84), GCTTATCCACCATGATCAAG (SEQ ID NO: 85), TGGAGAGGATGTGGAGAAAT (SEQ ID NO: 86), CTGCAGAGTGTTTTCCAACT (SEQ ID NO: 87), TCAGAGATTCAACTTCTTCC (SEQ ID NO: 88), TCTCTGAATAGACCAATAAC (SEQ ID NO: 89), GAATCTGGGTGCTCCTGTAT (SEQ ID NO: 90), CAAGTTGGAAAACACTCTGC (SEQ ID NO: 91), TAGATCCCATTTGTCAATTT (SEQ ID NO: 92), TGAAGCCCACTTGATCATGG (SEQ ID NO: 93), TCCAATTAAAAGACACAGAC (SEQ ID NO: 94), CAAAAGCCAAAATTGACAAA (SEQ ID NO: 95), GTATATACCCAGTAATGGGA (SEQ ID NO: 96), GAAATAAAGGGTATTCAATT (SEQ ID NO: 97), ACCCTCAGCTGCAGGTCTGT (SEQ ID NO: 98), CCAACTTACAAGGGATGTGA (SEQ ID NO: 99), ATTGAGAGTTTTTAGCATGA (SEQ ID NO: 100), TTTTTTGTTTTCCATTTGCT (SEQ ID NO: 101), TCTCTTCAAAGCTGTCAGAC (SEQ ID NO: 102), ATTCTTCCTACCCATGAGCA (SEQ ID NO: 103), AACACTTTTACACTGTTGGT (SEQ ID NO: 104), CTGTTTTTTCCCCATCTTTG (SEQ ID NO: 105), CAAACAACCCCATCAAAAAG (SEQ ID NO: 106), TTTCTAGTTCTAGATCCCTG (SEQ ID NO: 107), AGAACTTCCCCAATCTAGCA (SEQ ID NO: 108), TGTGAGATGGTATCTCATTG (SEQ ID NO: 109), TTTGAGTTCATTGTAGATTC (SEQ ID NO: 110), CCATGTTTAGTGCTTCCTTC (SEQ ID NO: 111), CAGTCTGAGATCAAACTGCA (SEQ ID NO: 112), TCAGTTTCCATGTAGTTGAG (SEQ ID NO: 113), TTAATCCAGTCTATCATTGT (SEQ ID NO: 114), GTCTAAAACACCAAAAGCAA (SEQ ID NO: 115), TGCCCTAAAAGAGCTCCTGA (SEQ ID NO: 116), TCACAGCCGAATTCTACCAG (SEQ ID NO: 117), AATGTCCAACAATGATAGAC (SEQ ID NO: 118), CTAGATTGGGGAAGTTCTCC (SEQ ID NO: 119), TTCTTTATTAGTCTTGCTAG (SEQ ID NO: 120), CCTCATAAAATGAGTTAGGG (SEQ ID NO: 121), GAAAAAATGCTCATCATCAC (SEQ ID NO: 122), AAGAATCAATATCGTGAAAA (SEQ ID NO: 123), GGTTTGCCAGTATTTTATTG (SEQ ID NO: 124), CTTCTCGAGGAGTATCTTTG (SEQ ID NO: 125), TTAATGATTGCCATTCTAAC (SEQ ID NO: 126), GGTAACCCGACCTTTCTCTC (SEQ ID NO: 127), AACAAAGCCTCCAAGAAATA (SEQ ID NO: 128), TAGCCCTTTGTCAGATGAGT (SEQ ID NO: 129), TAAACATGGAAAGGAACAAC (SEQ ID NO: 130), CTCCAACAGACCTGCAGCTG (SEQ ID NO: 131), GATGAGTTCATGTCCTTTGT (SEQ ID NO: 132), CAATCATGTCATCTGCAAAC (SEQ ID NO: 133), CTCTTTTAGGGCAGGCCTGG (SEQ ID NO: 134), TTTTGCATCAATGTTCATCA (SEQ ID NO: 135), CATGAACTCATCATTTTTTA (SEQ ID NO: 136), ATTTTGGAATAGGTGTGGTG (SEQ ID NO: 137), AAGTTCTGGCCAGGGCAATC (SEQ ID NO: 138), AATTCGGCTGTGAATCCATC (SEQ ID NO: 139), GTGGAGCCCACCACAGCTCA (SEQ ID NO: 140), TTTCATCCATGTCCCTACAA (SEQ ID NO: 141), AAAACAGAGATATAGATCAA (SEQ ID NO: 142), ATTGATCTATATCTCTGTTT (SEQ ID NO: 143), TAAAATCAGAGCAGAACTGA (SEQ ID NO: 144), AGTAGATAAAACCACAAAGA (SEQ ID NO: 145), GAACTACAAACCACTGCTCA (SEQ ID NO: 146), ATTGAATCTATAAATTACCT (SEQ ID NO: 147), AGTCAGTGTGGCGATTCCTC (SEQ ID NO: 148), TGTCTGTGCCCTGCCCCCAG (SEQ ID NO: 149), CGCCACACTGACTTCCACAA (SEQ ID NO: 150), TAGTTTTCCTTCTAACAGAC (SEQ ID NO: 151), AAATGTATATTCTGTTGATT (SEQ ID NO: 152), CTACTTTTGGTCTTTGATGA (SEQ ID NO: 153), AGACTCCCACACAATAATAA (SEQ ID NO: 154), GAAGCCCATCAGACTAACAG (SEQ ID NO: 155), GCCTCTGTAGGCTCCACCTC (SEQ ID NO: 156), TGGAGCCTACAGAGGCAGGC (SEQ ID NO: 157), TCCAAAATTGACCACATAGT (SEQ ID NO: 158), GATTTCTGCATTTCCATCTG (SEQ ID NO: 159), AACCTGAGAAAAACAAGCAA (SEQ ID NO: 160), TATTTCCTGAATTTGAATGT (SEQ ID NO: 161), GAACTCAGCTCTGCACCAAG (SEQ ID NO: 162), CAATACAGAGAAGTGCTTAA (SEQ ID NO: 163), CCCCATTGCTTGTTTTTCTC (SEQ ID NO: 164), TTACCAACCAAAAAGAGTCC (SEQ ID NO: 165), ATGCACACGTATGTTTATTG (SEQ ID NO: 166), CCTTTCAAAAAACCAGCTCC (SEQ ID NO: 167), AGACCAAATCTACGTCTGAT (SEQ ID NO: 168), CTTTAAGCACTTCTCTGTAT (SEQ ID NO: 169), AGTCTCCCATTATTATTGTG (SEQ ID NO: 170), ATACAAAAATTAATTCAAGA (SEQ ID NO: 171), GCAACCTACTCATCTGACAA (SEQ ID NO: 172), TAATGCCTAGGTTTTCTTCT (SEQ ID NO: 173), TGGTCTAAAATTCTCTTTTT (SEQ ID NO: 174), AGTCTCTTTGTAGGTCACTC (SEQ ID NO: 175), CTCTACAAGCCAGAAGAGAG (SEQ ID NO: 176), ACACCAATCAGACGTAGATT (SEQ ID NO: 177), GTGAAGAATGCAGAAGCCTC (SEQ ID NO: 178), CTTGAATTAATTTTTGTATA (SEQ ID NO: 179), TATTGCCTAGGTTTTCTTCT (SEQ ID NO: 180), GACAGCTTTGAAGAGAGCAG (SEQ ID NO: 181), AAAATTTTCTCCCATTCTGT (SEQ ID NO: 182), CCAGTTCCTCCTTGTACCTC (SEQ ID NO: 183), GGAAGAACATTCCATGCTCA (SEQ ID NO: 184), GAATGTATATTCTGTTGATT (SEQ ID NO: 185), ATCAGATAGTTGTAGATATG (SEQ ID NO: 186), TAAGATCAGAGCAGAACTGA (SEQ ID NO: 187), ATATTAACTTTAAATGTAAA (SEQ ID NO: 188), GCATTTTTTCATGTGTTTTT (SEQ ID NO: 189), TTCAAAAAATCAATGAATCC (SEQ ID NO: 190), CACCCTCCCAAGACTAAACC (SEQ ID NO: 191), AGATTTTGGGCTGAGACAAT (SEQ ID NO: 192), CACTCTCCCAAGACTAAACC (SEQ ID NO: 193), GTTTTCAACTTCTTTGCCTT (SEQ ID NO: 194), TATGTATACATGTGCCATGC (SEQ ID NO: 195), CACTAGGGAGTGCCAGACAG (SEQ ID NO: 196), ATCATCCTGATACCAAAGCC (SEQ ID NO: 197), GTGTGTCTCTGCACGTGAGA (SEQ ID NO: 198), TTTCTAGTTTATTTGCGTAG (SEQ ID NO: 199), GATTTCTGCATTTCCAACTG (SEQ ID NO: 200), TCTTTTATTTCCTTGAGCAG (SEQ ID NO: 201), TCACGTGCAGAGACACACAT (SEQ ID NO: 202), CACTCCAGACCCTGTTTGCC (SEQ ID NO: 203), ATATTAACCTTAAATGTAAA (SEQ ID NO: 204), CAGCATTTGCTTGTCTGTAA (SEQ ID NO: 205), GAGATCCGCTGTTAGTCTGA (SEQ ID NO: 206), CAGCATGATTTATAGTCCTT (SEQ ID NO: 207), CCCTACAAGCCAGAAGAGAG (SEQ ID NO: 208), ATACAAAAATCAATTCAAGA (SEQ ID NO: 209), ATTTAGCCCATTTACATTTA (SEQ ID NO: 210), TTTTTTGTTGTGTCTCTGCC (SEQ ID NO: 211), AGGGGTCAGGGACCCACTTG (SEQ ID NO: 212), TTTCTAGTTTATTTGCATAG (SEQ ID NO: 213), CTTGAATTGATTTTTGTATA (SEQ ID NO: 214), TGAATGTGTCCCAGAGATTC (SEQ ID NO: 215), AAAATTTTCTCCCATTTTGT (SEQ ID NO: 216), TGTTGTGTCTTTGTTCTCGT (SEQ ID NO: 217), AGCAAAGCCTCCAAGAAATA (SEQ ID NO: 218), AAGTTCTGGCCAGGGCAATT (SEQ ID NO: 219), ATTGAATCTGTAAATTACCT (SEQ ID NO: 220), AGACTCCCACACATTAATAA (SEQ ID NO: 221), CCATTCTCCCCATCACTTTC (SEQ ID NO: 222), GCTCTCTGTTTGTCTGTTAT (SEQ ID NO: 223), AGTCTCCCATTATTAATGTG (SEQ ID NO: 224), GTACAGATGGGTTTTTGGTG (SEQ ID NO: 225), TGCCTCCCAGTTAGGCTGCT (SEQ ID NO: 226), CCCACTCTCTTCTGGCTTGT (SEQ ID NO: 227), GCTGATGGAGCTGAAAACCA (SEQ ID NO: 228), ACTCCCTAGTGAGATGAACC (SEQ ID NO: 229), TTCAAAAAATTAATGAATCC (SEQ ID NO: 230), CACCTATGAGTGAGAATATG (SEQ ID NO: 231), ACATTCAAAGCAGTGTGTAG (SEQ ID NO: 232), AACATTCCATGCTCATGGGT (SEQ ID NO: 233), CTTCTCCTGCCTAATTGCCC (SEQ ID NO: 234), TTTGTTTACCTAAGCAAGCC (SEQ ID NO: 235), TCTTTTATTTCATTGAGCAG (SEQ ID NO: 236), ACTGCTCAATGAAATAAAAG (SEQ ID NO: 237), CCTGAAAGTGATGGGGAGAA (SEQ ID NO: 238), TAGTTTTCCTTCTAACAGTC (SEQ ID NO: 239), ATTTTGGCATGATTTTGCAG (SEQ ID NO: 240), CTTTGGTTCTGTTTATATGC (SEQ ID NO: 241), GACACAATAAAAAATGATAA (SEQ ID NO: 242), TTTCTTCCAGTTGATCGCAT (SEQ ID NO: 243), CTTTTCAAAAAACCAGCTCC (SEQ ID NO: 244), TTCACGTAGTTCTCGAGCCT (SEQ ID NO: 245), GAGCGCCTCTCCTCCTCCAA (SEQ ID NO: 246), TCAGATCTCCAGCTGCGTGC (SEQ ID NO: 247), AATTGAACAATGAGAACACA (SEQ ID NO: 248), ATGAATGAAATGAAGCGAGA (SEQ ID NO: 249), CAGTTTCTTCCTAGTCTCGA (SEQ ID NO: 250), CACCGCATATTCTCACTCAT (SEQ ID NO: 251), CTCAAAACCGCTCAACTACA (SEQ ID NO: 252), TCCACCCAGTTCGAGCTTCC (SEQ ID NO: 253), TGTTGTGTCTTTGTTCTCAT (SEQ ID NO: 254), GATGCGATCAACTGGAAGAA (SEQ ID NO: 255), GTACCAGTACCATGCTGTTT (SEQ ID NO: 256), AAAAAACAGAGCAGAAAAAC (SEQ ID NO: 257), CTTTGGTATCAGGATGATGC (SEQ ID NO: 258), AAAAAACAGAACAGAAAAAC (SEQ ID NO: 259), GTGCTTTACTTCCAACTATG (SEQ ID NO: 260), TAGATAAAACCACAAAGATG (SEQ ID NO: 261), TGACCCCCGAGCAGCCTAAC (SEQ ID NO: 262), AATTTGGCATGTTTTTGCAG (SEQ ID NO: 263), TAAAAGAGGATACAAACAAA (SEQ ID NO: 264), GCATTCAAAGCAGTGTGTAG (SEQ ID NO: 265), GAGGAACTGCGTTCCTTTGG (SEQ ID NO: 266), TTTGACGAGCTGAGAGAAGA (SEQ ID NO: 267), CTTTGGTATCAGAATGATGC (SEQ ID NO: 268), ATTCTTCCTATCCATGAGCA (SEQ ID NO: 269), TCCCTTTCCTAGTCAAAGAA (SEQ ID NO: 270), AAAACAGAGATATAGACCAA (SEQ ID NO: 271), CTTCTCCTGCCTGATTGCCC (SEQ ID NO: 272), TGGGAGTGACCCGATTTTCC (SEQ ID NO: 273), ATGTAAAGACCATCGAGACT (SEQ ID NO: 274), CCATTCTCCCCGTCACTTTC (SEQ ID NO: 275), TCACCATCATCAAAGACCAA (SEQ ID NO: 276), ATTATTATACTTTAAGTTTT (SEQ ID NO: 277), TCAATTTCAGAGCCTGTTAT (SEQ ID NO: 278), GCTCTCTGTTTGTCTGTTGT (SEQ ID NO: 279), AACGAGACAGAAAGTCAACA (SEQ ID NO: 280), CAGCATGATTTATAATCCTT (SEQ ID NO: 281), GATCAAATTACTCTGAGCTA (SEQ ID NO: 282), GATGCAATAAAAAATGATAA (SEQ ID NO: 283), CTTTGGCTCTGTTTATATGC (SEQ ID NO: 284), TGTCACCCCTTTCTTTGACT (SEQ ID NO: 285), GGTCAGGGACCCACTTGAGG (SEQ ID NO: 286), CTCTGAGACAAAACTTCCAG (SEQ ID NO: 287), CTGGCCTCATAAAATGAGTT (SEQ ID NO: 288), CTTCATCCATGTCCCTACAA (SEQ ID NO: 289), CCTGAAAGTGACGGGGAGAA (SEQ ID NO: 290), CACCTATGAGTGAGAACATG (SEQ ID NO: 291), TATTTCCTGAATCTGAACGT (SEQ ID NO: 292), AGGAGCCAAGATGGCCGAAT (SEQ ID NO: 293), AAGAATCAATATCATGAAAA (SEQ ID NO: 294), GCCATTGCCCAGGCTTGCTT (SEQ ID NO: 295), CGCAGCTGGAGATCTGAGAA (SEQ ID NO: 296), AATTGAACAATGAGATCACA (SEQ ID NO: 297), CAATCATGTCGTCTGCAAAC (SEQ ID NO: 298), AGACCGGAGCTGTTCCTATT (SEQ ID NO: 299), TATTTCCTGAATCTGAATGT (SEQ ID NO: 300), TGCCTTACAAGAGCTCCTGA (SEQ ID NO: 301), TTGGGAGAGTGTATGTGTCG (SEQ ID NO: 302), GGAAGGGGAACATCACACTC (SEQ ID NO: 303), TAAATGTGTCCCAGAGATTC (SEQ ID NO: 304), AGGTGTCAGTGTGCCCCTGC (SEQ ID NO: 305), TTAGGATTGACTTGGCGATG (SEQ ID NO: 306), TTCCAACAGACCTGCAGCTG (SEQ ID NO: 307), AACCTGACAAAAACAAGCAA (SEQ ID NO: 308), TATGTATACATGTGCCATGT (SEQ ID NO: 309), AACCTGACAAAAACAAGAAA (SEQ ID NO: 310), TTAATGATCGCCATTCTAAC (SEQ ID NO: 311), GTCCTTCGCCCACTTTTTGA (SEQ ID NO: 312), TCCAAAATTGACCACATACT (SEQ ID NO: 313), AGATTTTGGGCTGAGACGAT (SEQ ID NO: 314), TGAATGCGTCCCAGAGATTC (SEQ ID NO: 315), AGACTGGAGCTGTTCCTATT (SEQ ID NO: 316), ATACTATGCAGCCATAAAAA (SEQ ID NO: 317), GGGCAGACTGACACCTCACA (SEQ ID NO: 318), ATCCTTTGCCCACTTTTTGA (SEQ ID NO: 319), GGAAGGGGAATATCACACTC (SEQ ID NO: 320), ACGCAGTTCCTCACCAGCAA (SEQ ID NO: 321), AATGCTAGATGACGAGTTAG (SEQ ID NO: 322), GACAGCTTTGAAGAGAGTAG (SEQ ID NO: 323), GAGCTTTACTTCCAACTATG (SEQ ID NO: 324), CATGAACTCATCCTTTTTTA (SEQ ID NO: 325), CTAACTCGTCATCTAGCATT (SEQ ID NO: 326), ATCCAGCTTTGTTCCGTTGC (SEQ ID NO: 327), AGTCTCTTTGTAGGTCTCTA (SEQ ID NO: 328), CCATGTTTAGCGCTTCCTTC (SEQ ID NO: 329), CCCCATTGCTTGTTTTTGTC (SEQ ID NO: 330), GAGCTTTACTTCCAAGTATG (SEQ ID NO: 331), GACGCAATAAAAAATGATAA (SEQ ID NO: 332), CTAGGTTGGGGAAGTTCTCC (SEQ ID NO: 333), ATCAGATGGTTGTAGATGTG (SEQ ID NO: 334), CCCCATTTCTTGTTTTTGTC (SEQ ID NO: 335), GGGCACACTGACACCTCACA (SEQ ID NO: 336), CTACCTTTGGTCTTTGATGA (SEQ ID NO: 337), GACTAAAACACCAAAAGCAA (SEQ ID NO: 338), TTTCTAGTTCTAGATCCTTG (SEQ ID NO: 339), GAAAAAATGCTCACCATCAC (SEQ ID NO: 340), TTAGGATTGACTTGGCAATG (SEQ ID NO: 341), TTTTGTCTCAGAGGAGTACC (SEQ ID NO: 342), ACATTTAAAGCAGTGTGTAG (SEQ ID NO: 343), CCAGCTCCTCCTTGTACCTC (SEQ ID NO: 344), CTCTTGTAAGGCAGGCCTGG (SEQ ID NO: 345), GAGATCTGCTGTTAGTCTGA (SEQ ID NO: 346), GAGATCAGCTGTTAGTCTGA (SEQ ID NO: 347), AGGGCTCTGTTCTGTTCCAT (SEQ ID NO: 348), AACGAGACAGAAAGTTAACA (SEQ ID NO: 349), CTAAGCAAAAAGAACAAAGC (SEQ ID NO: 350), TTTTTCCTTCATTTCAACCT (SEQ ID NO: 351), CCAGCTCCTCTTTGTACCTC (SEQ ID NO: 352), GTTCTAATTTGATTGCACTG (SEQ ID NO: 353), AAGAATCAATATTGTGAAAA (SEQ ID NO: 354), GTGCAATCAAATTAGAACTC (SEQ ID NO: 355), AGCGTGAGCGACGCAGAAGA (SEQ ID NO: 356), TTTGACGAGTTGAGAGAAGA (SEQ ID NO: 357), CAAAAGACAAAATTGACAAA (SEQ ID NO: 358), CATCATTCTGATACCAAAGC (SEQ ID NO: 359), CAGCTTTGTTCTTTTTGCTT (SEQ ID NO: 360), TCTTTTGTTGCCATTGCTTT (SEQ ID NO: 361), GACTGTTGTGGGGTGGGGGG (SEQ ID NO: 362), GTGTGTCTCTGCATGTGAGA (SEQ ID NO: 363), TATTTACCCAGTAGTCATTC (SEQ ID NO: 364), TCACAGCCAAATTCTACCAG (SEQ ID NO: 365), GTCTTCTGCGTCGCTCACGC (SEQ ID NO: 366), CTCAAAACCACTCAACTACA (SEQ ID NO: 367), TTTCTCTTGCCTGATTGCCC (SEQ ID NO: 368), ACAATTTCAGCTCCTGTTAT (SEQ ID NO: 369), AGTTTGCCAGTATTTTATTG (SEQ ID NO: 370), CTAAAAACTCTCAATAAACT (SEQ ID NO: 371), AGAACTTCCCCAACCTAGCA (SEQ ID NO: 372), TTTCTAGTTTATTTGTGTAG (SEQ ID NO: 373), TTGGGAGGGTGTATGTGTCC (SEQ ID NO: 374), CAATGCAGAGAAGTCCTTAA (SEQ ID NO: 375), ACCTACTCAAGCCTCAGCAA (SEQ ID NO: 376), TCACATGCAGAGACACACAT (SEQ ID NO: 377), GAGCACCTCTCCTCCTCCAA (SEQ ID NO: 378), TCCCTTTCCGAGTCAAAGAA (SEQ ID NO: 379), CGGCAGCGAGGCTGGGGGAG (SEQ ID NO: 380), GTCCAAAACACCAAAAGCAA (SEQ ID NO: 381), GCATTTTTTCATGTGTCTGT (SEQ ID NO: 382), CATCATCCTGATACCAAAGC (SEQ ID NO: 383), CCCAATTAAAAGACACAGAC (SEQ ID NO: 384), ACAATTTCAGATCCTGTTAT (SEQ ID NO: 385), TCACAGCTGAATTCTACCAG (SEQ ID NO: 386), TTACCAACCAAAAAAAGTCC (SEQ ID NO: 387), GTGTGTCTCTGCACATGAGA (SEQ ID NO: 388), GCCTCTGTAGACTCCACCTC (SEQ ID NO: 389), AGGTGTCAGTCTGCCCCTAC (SEQ ID NO: 390), ACTGACCTGCGCCCACTGTC (SEQ ID NO: 391), TCATGTGCAGAGACACACAT (SEQ ID NO: 392), GGTAACCTGACCTTTCTCTC (SEQ ID NO: 393), GCAATCTACTCATCTGACAA (SEQ ID NO: 394), CACCGCATGTTCTCACTCAT (SEQ ID NO: 395), TAGCAATCAGCGAGACTCCG (SEQ ID NO: 396), AAATGAAGGAAAAAATGTTA (SEQ ID NO: 397), ACAAAGAGAATAAAATACCT (SEQ ID NO: 398), TTAATCCAGTCTATCATTGA (SEQ ID NO: 399), GTAAATTAGTTCAACCATTG (SEQ ID NO: 400), AGGACCCTCCGAGCCAGGTG (SEQ ID NO: 401), CGTCACCCCTTTCTTTGACT (SEQ ID NO: 402), ATGAGTTCATGTCCTTTGTA (SEQ ID NO: 403), ACAATTTCAGAGCCTGTTAT (SEQ ID NO: 404), CCATTCTCCCTGTCACTTTC (SEQ ID NO: 405), GATCTGTCTAATATTGACAG (SEQ ID NO: 406), AATGTCCATCAATGATAGAC (SEQ ID NO: 407), CTCGGAGGGTCCTACGCCCA (SEQ ID NO: 408), TTTAAGTTCTTTGTAGATTC (SEQ ID NO: 409), CACCAGCAACAGAACAAAGC (SEQ ID NO: 410), TCATCTCACACCAGTTAGAA (SEQ ID NO: 411), AGACCAAATCTACATCTGAT (SEQ ID NO: 412), GAGATCCACTGTTAGTCTGA (SEQ ID NO: 413), TGACCCAGCAATCCCATTAC (SEQ ID NO: 414), ATCCAGCTTTGTTCCATTGC (SEQ ID NO: 415), GGAAGGGGAACATCACACAC (SEQ ID NO: 416), GCATTTTTTCATGTGTCTTT (SEQ ID NO: 417), CTCAAAACTGCTCAACTACA (SEQ ID NO: 418), TGCCTCCCAGTTAGGCTACT (SEQ ID NO: 419), TTTATTATACTTTAAGTTTT (SEQ ID NO: 420), CCTGATGGAGCTGAAAACCA (SEQ ID NO: 421), GTCCAGCTTTGTTCCATTGC (SEQ ID NO: 422), GTCCTTTGCCCACTTTTTGA (SEQ ID NO: 423), ACACCAATCAGATGTAGATT (SEQ ID NO: 424), CAGCTCCATCAGGTCCTTTA (SEQ ID NO: 425), GAGTGCCTCTCCTCCTCCAA (SEQ ID NO: 426), AGATTTTGGGCTGAGATGAT (SEQ ID NO: 427), AATTCAGCTGTGAATCCATC (SEQ ID NO: 428), TATTGGGTGCATATATATTT (SEQ ID NO: 429), CCTGAAAGTGACAGGGAGAA (SEQ ID NO: 430), AAAACAACCCCATCAAAAAG (SEQ ID NO: 431), TTAATGATCACCATTCTAAC (SEQ ID NO: 432), CTACCAACCAAAAAAAGTCC (SEQ ID NO: 433), CTGAAGAGTGTTTTCCAACT (SEQ ID NO: 434), CTTCTCAAGGAGTATCTTTG (SEQ ID NO: 435), CAGACTAACAGCTGATCTCT (SEQ ID NO: 436), CACCGTGCGCGAGCCGAAGC (SEQ ID NO: 437), CTTCATCCATGTCCCTGCAA (SEQ ID NO: 438), GGCAATGCCTCGCCCTGCTT (SEQ ID NO: 439), ATTGAATCTATAAATTACTT (SEQ ID NO: 440), CTCTTTGTAGCAATTGTGAA (SEQ ID NO: 441), CTTCTTGAGGAGTATCTTTG (SEQ ID NO: 442), TTTTTGCATCGATGTTCATC (SEQ ID NO: 443), CAGCTCCATCAGGTCATTTA (SEQ ID NO: 444), GAGTGAGAACATGCAGTGTT (SEQ ID NO: 445), AGTCAGGAAACAACAGATGC (SEQ ID NO: 446), CGATAGTTTGCTGAGAATGA (SEQ ID NO: 447), AATTTTCAGCTTTTCTGCTC (SEQ ID NO: 448), ATACCCAGTAATGGGATTGC (SEQ ID NO: 449), GAGGAGCTGCGTTCCTTTGG (SEQ ID NO: 450), AATTGAACAATGAGAACACT (SEQ ID NO: 451), AATGCTAAATGACGAGTTAA (SEQ ID NO: 452), TTTTTTGCTTTCCATTTGCT (SEQ ID NO: 453), ATGAATGAAATGAAGTGAGA (SEQ ID NO: 454), ATTCTCAGCAAACTATCGCA (SEQ ID NO: 455), CAAGTTGGAAAACACTCTTC (SEQ ID NO: 456), ATCATTCTGATACCAAAGCC (SEQ ID NO: 457), ACAACCTACTCATCTGACAA (SEQ ID NO: 458), TAGCATCAACATCAACAAAA (SEQ ID NO: 459), CAGTTTCTTCCTAGCCTTGA (SEQ ID NO: 460), AATTTGGCTGTGAATCCATC (SEQ ID NO: 461), TTTGTGGTTTTATCTACCTT (SEQ ID NO: 462), GCTGATGGAGCTGAAAGCCA (SEQ ID NO: 463), TTAACTCGTCATTTAGCATT (SEQ ID NO: 464), TGATAGTTTGCTGAGAATGA (SEQ ID NO: 465), GTTTTGCCAGTATTTTATTG (SEQ ID NO: 466), ATCCAGCTTTGTTCTGTTGC (SEQ ID NO: 467), AAGAACTTGCTTTATGAATC (SEQ ID NO: 468), CCTGACCCCTTGCGCTTCCC (SEQ ID NO: 469), TTGGGAGGGTGTATGTGTCG (SEQ ID NO: 470), CAGACTAACAGCAGATCTCT (SEQ ID NO: 471), TTGCTGCCTGATCCTTCCTC (SEQ ID NO: 472), TCTAAAATTGACCACATAAT (SEQ ID NO: 473), CTCAAAGCCGCTCAACTACA (SEQ ID NO: 474), ATACAAAAATTAACTCAAGA (SEQ ID NO: 475), ACAGACGGCACCTGGAAAAT (SEQ ID NO: 476), TCACCAACATCAAAGACCAA (SEQ ID NO: 477), GTCCAGCTTTGTTCCGTTGC (SEQ ID NO: 478), ATACCCAGGCAAACAGGGTC (SEQ ID NO: 479), CGCCACACTGTCTTCCACAA (SEQ ID NO: 480), CTTCCAATACTATGTTGAAT (SEQ ID NO: 481), AGCAGCCGGGAAGCTCGAAC (SEQ ID NO: 482), ACTCCTATTCAACATAGTAT (SEQ ID NO: 483), GTGTTTTACTTCCAATTATG (SEQ ID NO: 484), AAAGGGATCAATTCAACAAG (SEQ ID NO: 485), AATGAGACAGAAAGTTAACA (SEQ ID NO: 486), GACGGACGCACCTGGAAAAT (SEQ ID NO: 487), CTTGAGTTAATTTTTGTATA (SEQ ID NO: 488), AAAATTTTCTCCCATGTTGT (SEQ ID NO: 489), GAAAATCCTCAATAAAATAC (SEQ ID NO: 490), TTTCTCCTGCCTGATTGCCC (SEQ ID NO: 491), ATATTAGCCCTTTGTCAGAT (SEQ ID NO: 492), GGTAACCCAACCTTTCTCTC (SEQ ID NO: 493), AAACTATCATCAGAGTGAAC (SEQ ID NO: 494), AAAACAGATATATAGACCAA (SEQ ID NO: 495), TGCCTCACCTGGGAAGCGCA (SEQ ID NO: 496), TGCCATTGCTTTTGGTGTTT (SEQ ID NO: 497), AGGAAGATCTACCAAGCCAA (SEQ ID NO: 498), TGCCTTTTTTTGTTTTCCAT (SEQ ID NO: 499), ATTCTCAGCAAACTATCACA (SEQ ID NO: 500), CTGGACTTTTTTTGGTTGGT (SEQ ID NO: 501), CAGTTTCTTCCTAGCCTCGA (SEQ ID NO: 502), TAGGAACACTTTTACACTGT (SEQ ID NO: 503), ACGAGACTATATCCCACACC (SEQ ID NO: 504), GAATATTGCGCTTTTCAGAC (SEQ ID NO: 505), TTTGAGTTCTTTGTAGATTC (SEQ ID NO: 506), ATGCACATGTATGTTTATTG (SEQ ID NO: 507), TCAGGGATTCAACTTCTTCC (SEQ ID NO: 508), ATGCACACATATGTTTATTG (SEQ ID NO: 509), GCAGGGCATAGCTGAACAAA (SEQ ID NO: 510), TCAGATCTCCAGCTGCATGC (SEQ ID NO: 511), AATAACAAGTTCTGAAATTG (SEQ ID NO: 512), TGTGAGATGATATCTCATAG (SEQ ID NO: 513), ATCATCCTGATACCAAAACC (SEQ ID NO: 514), AGGCCTCTGTTCTGTTCCAT (SEQ ID NO: 515), TGACCCCCGAGTAGCCTAAC (SEQ ID NO: 516), GCCCACGCCTATGTCCTGAA (SEQ ID NO: 517), TCAATTTCAGAACTTGTTAT (SEQ ID NO: 518), TACCATTCAGGACATAGGCG (SEQ ID NO: 519), CACCACATGTTCTCACTCAT (SEQ ID NO: 520), AGGACCCTCTGAGCCAGGTG (SEQ ID NO: 521), CATAATTGTCAGATTCACCA (SEQ ID NO: 522), and GAAGACCTTAAATGACCTGA (SEQ ID NO: 523). Sequences are presented from 5' to 3'.

Provided herein are methods of obtaining sequences from a desired location, such as a LINE element. In some cases, the LINE element comprises a nucleotide polynucleotide having comprising SEQ ID NO: 1. In some cases, a portion of the LINE element is targeted, the portion having a sequence comprising SEQ ID NO: 2. In some cases, the LINE element is targeted using a guide RNA having a sequence comprising SEQ ID NO: 3.

Provided herein are methods of obtaining sequences adjacent to a desired location, such as an Alu element. In some cases, the Alu element comprises an Alu-Y element having a sequence comprising SEQ ID NO: 4.

In some cases, the desired genomic location is amplified by a DNA polymerase, for example a strand-displacing DNA polymerase. In some cases a DNA polymerase is used to amplify the desired genomic location. In some cases the DNA polymerase requires a primer sequence to be included in the tag, for example a DNA primer or an RNA primer.

Tags are inserted into a sample of genomic DNA by genome editing or gene targeting methods known by skill in the art. In some cases, tags are inserted into the genomic DNA of a cell, such as an isolated cell from a patient or a cultured cell. In some cases, tags are inserted into genomic DNA that has been isolated from a cell or tissue, such as a cell or tissue sample from a patient.

Genome editing or gene targeting technology is understood by one of skill in the art and includes methods such as homologous recombination, clustered regularly-interspaced short palindromic repeats (CRISPR), (transcription activator-like effector nucleases (TALENS), Zinc finger nucleases, transposons, and other methods. The result of any of these methods is a specific insertion into the genome of one or more nucleic acid tags comprising at least one promoter sequence.

In methods described herein, the tag to be inserted into the desired genomic location is a nucleic acid. Depending on the exact mechanism of genomic targeting the nucleic acid tag is a RNA or a DNA. In some cases the nucleic acid tag is a RNA/DNA hybrid. Nucleic acid tags are prepared for a gene targeting reaction by methods known by one of skill in the art. In some cases, tags are synthesized by nucleic acid synthesizers. In some cases, tags are prepared by recombinant DNA technology. An RNA nucleic acid tag, in some cases is transcribed from a plasmid. Depending on the method of insertion, the sequence complementary to the desired genomic location will vary to accommodate the method. In some cases, CRISPR requires a PAM sequence that must be located in the genomic location to be targeted.

In some cases, CRISPR is used to insert the tagged nucleic acid sequence into a specific location in the genomic DNA sample. CRISPR generally uses two components, a guide RNA (gRNA) and a non-specific CRISPR-associated endonuclease (Cas9). In some cases, a CRISPR gRNA requires a scaffold sequence for Cas9 binding and a targeting sequence of about 20 nucleotides containing the tag and the genomic DNA sequence to be modified.

Also disclosed herein are methods comprising insertion of a tagged nucleic acid sequence into a CRISPR targeted site in a DNA sample. In these methods, a CRISPR/CAS nuclease creates a double stranded break at a targeted site in the genome. An exonuclease is then added to the reaction mixture which degrades one strand of the double stranded break creating a sticky end with a specific nucleic acid sequence. Alternatively, a second targeted CRISPR/CAS nuclease is used to create a sticky end intead of the exonuclease. The tagged nucleic acid polynucleotide comprises a single stranded nucleic acid having portion comprising a transcriptional promoter such as a T7 promoter and a portion comprising a nucleic acid sequence complementary to the sticky end. Optionally, the tagged nucleic acid polynucleotide comprises a portion that is self-complementary allowing the tagged nucleic acid to form a hairpin.

### Linear Amplification of Nucleic Acids

Methods provided herein allow for obtaining precise and accurate sequence information from nucleic acid molecules with sequences known by one of skill in the art to be difficult to sequence. Methods herein use targeted nucleic acid sequences that are amplified in a linear fashion directly from the starting genomic DNA template. As would be appreciated by one of skill in the art, genomic regions that are difficult to sequence have characteristics which are known to have increased rates of replication errors such as insertions, deletions, and substitutions, caused by DNA polymerases, such as Taq polymerase. In amplification methods such as PCR, these errors are passed on with each round of amplification, creating amplification-specific sequencing errors that do not reflect the original template.

Methods disclosed herein include linear amplification, that is creation of additional nucleic acid molecules identical to the original genomic DNA template, synthesized directly from the original template. In some cases, linear amplification is achieved using transcription, for example in vitro transcription of RNA from a specific genomic DNA location that has been specifically tagged to contain a promoter sequence as described herein. Suitable RNA polymerases include but are not limited to T7 RNA polymerase, T3 RNA polymerase SP6 RNA polymerase, RNA polymerase I, RNA polymerase II, RNA polymerase III RNA polymerase IV. In some cases, RNA is transcribed by a RNA polymerase V, single subunit RNA polymerase. In some cases, the in vitro transcription reaction requires one or more ribonucleotides (ATP, GTP, UTP, and CTP) and buffers suitable to the RNA polymerase.

### Purification of linearly amplified nucleic acids

Methods disclosed herein provide for purification of the linearly amplified nucleic acid from the genomic DNA template. In some instances, the method of purification is an enzymatic method whereby the genomic DNA template is digested using one or more DNases. Alternately, the method of purification is an affinity based purification whereby the resulting amplified nucleic acid is labeled and a reagent such as an antibody binds to the labeled amplified nucleic acids and the unbound genomic DNA template is washed away from the bound amplified nucleic acids. The method of purification is also contemplated to be a fluorescence based sorting purification, whereby fluorescently labeled amplified nucleic acids are sorted away from unlabeled genomic DNA template. Further purification methods include wherein the amplified nucleic acids are purified from the amplification reaction after each round of amplification. In further methods, the amplified nucleic acids are purified after the amplification reaction is complete.

Methods disclosed herein provide for an amplified nucleic acid that is an RNA. In instances where it is desired to have a DNA sample for downstream steps in methods, a DNA copy is made from the RNA using one or more reverse transcriptase enzymes. Optionally, the RNA is polyadenylated prior to treatment with reverse transcriptase. Alternately, the reverse transcriptase uses an oligo dT for priming the reverse transcriptase reaction. The reverse transcriptase is also contemplated to use a gene specific primer for priming the reverse transcriptase reaction. The reverse transcriptase optionally uses random hexamer primers for priming the reverse transcriptase reaction. It is also contemplated that, the reverse transcriptase uses a buffer and deoxyribonucleotides.

### Border-adjacent libraries and sequence databases

Disclosed herein are nucleic acid libraries comprising molecules comprising mobile element edges or borders and mobile element adjacent genomic or other non-mobile element sequence, paired in nucleic acid molecules such that library constituent molecules have both a mobile element border and mobile element adjacent sequence so as to identify the mobile element location within a genome or other nucleic acid source.

As discussed elsewhere in the present disclosure, libraries consistent with the disclosure comprise molecules generated through iterative linear amplification of products direct from a sample template. Accordingly, such libraries do not suffer from differential amplification artifacts that arise from chain-reaction based amplification methods that involve early rounds of amplification to yield products being used as templates in subsequent amplification reactions, resulting in early amplification errors being propagated in later products. Through linear amplification, particularly but not exclusively through an RNA intermediary, amplification products are uniquely and distinctly derived directly from the template. Any error in generation of a particular amplification product is not propagated in subsequent reactions, because the amplification products do not serve as templates for chain reaction amplification in library generation. Errors in amplification occur, but are independent of one another, individually rare, and easily recognized by comparison to related amplification products of the same template.

Often, libraries consistent with the disclosure herein are derived from genomic DNA, but other nucleic acid sources are also contemplated. Libraries consistent with the disclosure herein often share a common element of being enriched for mobile element border and border adjacent sequence containing molecules relative to a genomic or other nucleic acid sample from which the libraries are derived. That is, relative to a genomic sample, a greater number of nucleic acid molecules comprise both a mobile element border and border-adjacent sequence, or a greater proportion of the total sequence of the library is in proximity to a mobile element border.

Libraries herein comprise at least 100, 200, 500, 1,000, 5,000, 10,000, 20,000, 50,000, 100,000, 200,000, 500,000, 1,000,000, or more than 1,000,000 nucleic acid molecules. Some libraries disclosed herein comprise at least 10x, 20x, 50x, 100x, 200x, 500x, 1,000x, 2,000x, 5,000x, 10,000x, 20,000x, 50,000x, 100,000x or greater than 100,000x the number of chromosomes in a haploid complement of chromosomes of a nucleic acid sample.

In some cases 10%, 20%, 30%, 40%, 50%, 60%, 70%, 80%, 85%, 90%, 91%, 92%, 93%, 94%, 95%, 96%, 97%, 98%, 99%, or more than 99%, such as 99.5%, 99.6%, 99.7%, 99.8%, 99.9%, about 100%, or 100% of the library constituents comprise both a mobile element border and border-adjacent sequence.

'Sequence in proximity to a mobile element border' is variously understood to refer to sequence (measured at a given base position) for which a mobile element border is located no more than 20kb, 15kb, 10kb, 9kb, 8kb, 7kb, 6kb, 5kb, 4kb, 3kb, 2kb, 1kb, 900 bases, 800 bases, 700 bases, 600 bases, 500 bases 400 bases, 300 bases, 200 bases, 100 bases, or less than 100 bases from the sequence.

In libraries consistent with the disclosure herein, sequence in proximity to a mobile element border represents a greater proportion of the overall sequence of the library than in a related sample such as a genomic sample from which the library is generated. In some cases 10%, 20%, 30%, 40%, 50%, 60%, 70%, 80%, 85%, 90%, 91%, 92%, 93%, 94%, 95%, 96%, 97%, 98%, 99%, or more than 99%, such as 99.5%, 99.6%, 99.7%, 99.8%, 99.9%, about 100%, or 100% of the library constituents comprise sequence in proximity to a mobile element border.

Also disclosed herein are nucleic acid libraries comprising molecules comprising repetitive region edges or borders and repetitive region adjacent genomic or other non-repetitive region sequence, paired in nucleic acid molecules such that library constituent molecules have both a repetitive region border and repetitive region adjacent sequence so as to identify the repetitive region location within a genome or other nucleic acid source.

Often, libraries consistent with the disclosure herein are derived from genomic DNA, but other nucleic acid sources are also contemplated. Libraries consistent with the disclosure herein often share a common element of being enriched for repetitive region border and border adjacent sequence containing molecules relative to a genomic or other nucleic acid sample from which the libraries are derived. That is, relative to a genomic sample, a greater number of nucleic acid molecules comprise both a repetitive region border and border-adjacent sequence, or a greater proportion of the total sequence of the library is in proximity to a repetitive region border.

In some cases 10%, 20%, 30%, 40%, 50%, 60%, 70%, 80%, 85%, 90%, 91%, 92%, 93%, 94%, 95%, 96%, 97%, 98%, 99%, or more than 99%, such as 99.5%, 99.6%, 99.7%, 99.8%, 99.9%, about 100%, or 100% of the library constituents comprise both a repetitive region border and border-adjacent sequence.

'Sequence in proximity to a repetitive region border' is variously understood to refer to sequence (measured at a given base position) for which a repetitive region border is located no more than 20kb, 15kb, 10kb, 9kb, 8kb, 7kb, 6kb, 5kb, 4kb, 3kb, 2kb, 1kb, 900 bases, 800 bases, 700 bases, 600 bases, 500 bases 400 bases, 300 bases, 200 bases, 100 bases, or less than 100 bases from the sequence.

In libraries consistent with the disclosure herein, sequence in proximity to a repetitive region border represents a greater proportion of the overall sequence of the library than in a related sample such as a genomic sample from which the library is generated. In some cases 10%, 20%, 30%, 40%, 50%, 60%, 70%, 80%, 85%, 90%, 91%, 92%, 93%, 94%, 95%, 96%, 97%, 98%, 99%, or more than 99%, such as 99.5%, 99.6%, 99.7%, 99.8%, 99.9%, about 100%, or 100% of the library constituents comprise sequence in proximity to a repetitive region border.

Libraries consistent with the disclosure comprise fragments that comprise both a repetitive region border and border-adjacent sequence and that span at least 50, 100, 200, 300, 400, 500, 600, 700, 800, 900, 1,000, 1,500, 2,000, 2,500, 3,000, 3,500, 4,000, 5,000, 6,000, 7,000, 8,000, 9,000, 10,000, 20,000 or greater than 20,000 bases. Some libraries consistent with the disclosure herein comprise a plurality of fragments that comprise both a repetitive region border and border-adjacent sequence, such that said plurality of fragments exhibit a mean fragment size of 50, 100, 200, 300, 400, 500, 600, 700, 800, 900, 1,000, 1,500, 2,000, 2,500, 3,000, 3,500, 4,000, 5,000, 6,000, 7,000, 8,000, 9,000, 10,000, 20,000 or greater than 20,000 bases. Some libraries consistent with the disclosure herein comprise a plurality of fragments that comprise both a repetitive region border and border-adjacent sequence, such that said plurality of fragments exhibit a median fragment size of 50, 100, 200, 300, 400, 500, 600, 700, 800, 900, 1,000, 1,500, 2,000, 2,500, 3,000, 3,500, 4,000, 5,000, 6,000, 7,000, 8,000, 9,000, 10,000, 20,000 or greater than 20,000 bases.

Libraries as disclosed herein comprise RNA or DNA. Some libraries arise from RNA transcription directed by an RNA polymerase promoter that is inserted, such as selectively inserted, into a mobile element or other repetitive region in a nucleic acid sample such as a genomic DNA sample. Some libraries comprise molecules generated from this transcription, including molecules optionally comprising RNA polymerase promoter sequence, repetitive region such as mobile element sequence, and mobile element or other repetitive region adjacent sequence such as adjacent genomic sequence. Some libraries comprise said RNA arising from RNA transcription directed by an inserted RNA polymerase promoter. Alternately, many libraries comprise DNA generated through revers transcription of a population of RNA molecules generated as described above.

Libraries as disclosed herein are generated from templates such as genomic nucleic acid samples, and are reflective of genomic sequence adjacent to repetitive regions in the samples. In some embodiments, the repetitive elements comprise mobile elements such as Alu repeats or transposons that are observed to relocate to various positions throughout a genome. This relocation or transposition is often specific to a cell or population of cells in an organism from which a genomic sample is derived. Alternatively, libraries are reflective of genomic sequence adjacent to a selected genomic region. Libraries are derived from samples such as genomic DNA samples from a population of cells such as tumor cells or healthy cells. In some cases a library is generated from a genomic DNA derived from a single cell.

In particular, some mobile element insertion events or transposition events are implicated in lineage-specific cell defects, such as cell cycle or cell growth regulatory defects as are often implicated in cancer cell lines. To facilitate identification of mobile element insertion events that are implicated in cancer, libraries are generated from templates such as genomic nucleic acid samples obtained from cancer or tumor cells or tissues, either alone or in combination with generation of libraries from noncancerous cells or tissues.

Libraries consistent with the disclosure herein are optionally sequenced so as to determine sequence adjacent to a repetitive or repeated region, such as a mobile element border or repeat adjacent sequence of a repetitive locus such as the HLA locus. Alternatively, libraries consistent with the disclosure herein are optionally sequenced as to determine sequence adjacent to a selected genomic region. Library constituents are sequenced using any number of sequencing approaches disclosed elsewhere herein or otherwise known to one of skill in the art, such as shotgun sequencing, next generation sequencing by synthesis approaches, long molecule sequencing such as PacBio, BioNano or Oxford Nanopore sequencing.

A sequence database generated hereby comprises nucleic acid sequences of a library consistent with the disclosure herein or practice of a method consistent with the disclosure herein. In some cases 10%, 20%, 30%, 40%, 50%, 60%, 70%, 80%, 85%, 90%, 91%, 92%, 93%, 94%, 95%, 96%, 97%, 98%, 99%, or more than 99%, such as 99.5%, 99.6%, 99.7%, 99.8%, 99.9%, about 100%, or 100% of the database constituents comprise both a repeat element such as a mobile element sequence and border-adjacent sequence.

In databases consistent with the disclosure herein, sequence in proximity to a mobile element border represents a greater proportion of the overall sequence of the library than in a related sample such as a genomic sample from which the library is generated. In some cases 10%, 20%, 30%, 40%, 50%, 60%, 70%, 80%, 85%, 90%, 91%, 92%, 93%, 94%, 95%, 96%, 97%, 98%, 99%, or more than 99%, such as 99.5%, 99.6%, 99.7%, 99.8%, 99.9%, about 100%, or 100% of the database constituents comprise sequence in proximity to a repeat element such as a mobile element border.

Databases consistent with the disclosure comprise sequences that comprise both a repetitive region border and border-adjacent sequence and that span at least 50, 100, 200, 300, 400, 500, 600, 700, 800, 900, 1,000, 1,500, 2,000, 2,500, 3,000, 3,500, 4,000, 5,000, 6,000, 7,000, 8,000, 9,000, 10,000, 20,000 or greater than 20,000 bases. Some databases consistent with the disclosure herein comprise a plurality of sequences that comprise both a repetitive region border and border-adjacent sequence, such that said plurality of sequences exhibit a mean sequence length of 50, 100, 200, 300, 400, 500, 600, 700, 800, 900, 1,000, 1,500, 2,000, 2,500, 3,000, 3,500, 4,000, 5,000, 6,000, 7,000, 8,000, 9,000, 10,000, 20,000 or greater than 20,000 bases. Some databases consistent with the disclosure herein comprise a plurality of sequences that comprise both a repetitive region border and border-adjacent sequence, such that said plurality of fragments exhibit a median fragment size of 50, 100, 200, 300, 400, 500, 600, 700, 800, 900, 1,000, 1,500, 2,000, 2,500, 3,000, 3,500, 4,000, 5,000, 6,000, 7,000, 8,000, 9,000, 10,000, 20,000 or greater than 20,000 bases.

Databases as disclosed herein are in some cases completely sequenced, such that database entries comprise end-to-end sequence information for library molecules from which they are generated. Alternately, in some cases some or all of a database's entries comprise paired reads, such that one member of a paired read comprises repeat element sequence such as mobile element sequence, while the other member of a paired read comprises repeat adjacent sequence, such that in some cases a mobile element border location is inferred without actually sequencing across the border of the mobile element.

### Sequencing of linearly amplified nucleic acids

Methods disclosed herein optionally comprise sequencing the linearly amplified nucleic acids, such as those generated pursuant to the production of libraries as disclosed herein. In some cases, the methods comprise annealing an oligonucleotide required for sequencing to the linearly amplified nucleic acids. In some cases, the sequencing comprises ligating an oligonucleotide required for sequencing to the linearly amplified nucleic acids. In some cases, the methods comprise utilizing the adapter sequence or portion thereof to sequence the linearly amplified nucleic acids.

Various methods of nucleic acid sequencing are well-known and described in the art. The methods disclosed herein are consistent with a wide range of sequencing technologies.

Determination of the sequence of an linearly amplified nucleic acid is contemplated herein to be performed using a sequencing method selected from a variety of sequencing methods including, but not limited to, ion detection technology, DNA nanoball technology, nanopore-based sequencing technology, sequencing by hybridization (SBH), sequencing by ligation (SBL), quantitative incremental fluorescent nucleotide addition sequencing (QIFNAS), stepwise ligation and cleavage, fluorescence resonance energy transfer (FRET), molecular beacons, TaqMan reporter probe digestion, pyrosequencing, fluorescent in situ sequencing (FISSEQ), FISSEQ beads, wobble sequencing, multiplex sequencing, polymerized colony (POLONY) sequencing; nanogrid rolling circle sequencing (ROLONY), allele-specific oligo ligation assays (e.g., oligo ligation assay (OLA), single template molecule OLA using a ligated linear probe and a rolling circle amplification (RCA) readout, ligated padlock probes, and/or single template molecule OLA using a ligated circular padlock probe and a rolling circle amplification (RCA) readout) and the like. Optionally, high-throughput sequencing methods such as cyclic array sequencing using platforms such as Roche 454, Illumina Solexa, ABI-SOLiD, ION Torrents, Complete Genomics, Pacific Bioscience, Helicos, Polonator platforms, are utilized.

Determination of the sequence of a linearly amplified nucleic acid is optionally contemplated to be performed by a next-generation sequencing (NGS) method. NGS applies to genome sequencing, genome resequencing, transcriptome profiling (RNA-Seq), DNA-protein interactions (ChIP-sequencing), and epigenome characterization. Contemplated methods disclosed herein comprise NGS methods selected from, but are not limited to, massively parallel signature sequencing (MPSS), Polony sequencing, 454 pyrosequencing, Ion Torrent semiconductor sequencing, Heliscope single molecule sequencing, single molecule real time (SMRT) sequencing and microfluidic Sanger sequencing.

### Mapping of Genomic Elements

Methods disclosed herein are optionally contemplated to comprise mapping a genomic element, for example a mobile genetic element using the sequences obtained from the methods provided herein. Alternately, the method comprises insertion of a tag, such as a nucleic acid tag, comprising a promoter, such as a T7 promoter, into the genomic element using genomic DNA editing technology, such as CRISPR. An RNA molecule is transcribed, from the inserted tag allowing for linear amplification of RNA having the same sequence as the DNA adjacent to the genomic element. The sequence obtained from the linearly amplified nucleic acid allows one to find the corresponding location by comparing the sequence obtained to the sequence available for the reference genome, for example a human genome sequence, in the genome and thereby map the insertion of the genomic element. Optionally, the insertion of the genomic element maps to the coding sequence of a gene. Alternately, the insertion of the genomic element maps to an intron of a gene. It is also contemplated that the insertion of the genomic element maps to a promoter or enhancer sequence of a gene. Optionally, the insertion of the genomic element maps to a 5' or 3' untranslated region of a gene.

Insertion of a genomic element, for example a mobile genetic element near or into a gene, for example in the coding sequence, in an intron, into a promoter or enhancer, or into a 5' or 3' untranslated region, often causes disruption of the function of the gene. Disruption of gene function by insertion of a genetic element, for example a mobile genetic element, can occur by any one of a number of mechanisms known to one of skill in the art. Often, gene function is disrupted by insertion into the coding sequence, thereby disrupting or otherwise changing the amino acid sequence of the gene. Alternately, gene function is disrupted by insertion into an intron or 5' or 3' untranslated region, thereby affecting gene expression downstream of transcription, for example RNA splicing, RNA transport, and RNA translation. Optionally, gene function is disrupted by insertion into a promoter or enhancer element, thereby affecting assembly of gene regulatory proteins onto the chromatin and transcription of the gene.

Insertion of a genomic element and thereby disruption of function of one or more genes, often is a cause of disease. Optionally, the disease is a cancer. Alternatively, the disease is aging. It is contemplated herein that mapping the insertion of a genomic element informs a health care provider information, for example diagnostic information, that assists the healthcare provider in making decisions regarding treatment of the individual. Optionally, mapping the insertion of a genetic element provides a diagnosis for cancer. Alternatively, mapping the insertion of a genomic element provides a diagnosis for aging.

In some cases, provided herein are methods of diagnosing cancer in a subject, comprising obtaining a sample from the subject, isolating genomic DNA from the sample, contacting the genomic DNA with a composition that inserts a tag polynucleotide comprising a T7 promoter into a genomic element of the genomic DNA sample, performing in vitro transcription with a T7 RNA polymerase to obtain an RNA sample that has been linearly amplified from the genomic DNA, treating the sample with DNase to remove the genomic DNA from the sample, polyadenylating the RNA sample and reverse transcribing the RNA sample to obtain a cDNA, treating the sample with RNaseH to remove the RNA from the sample, and subjecting the resulting cDNA sample to DNA sequencing using a Next generation sequencing method resulting in a sequence that is adjacent to the genomic element. The sequence that is adjacent to the genomic element searched in a bioinformatics database such as BLAST to determine the location of the insertion of the genomic element and thereby determine the identity of any genes near the insertion.

### Sequencing Challenging Genomic Regions

Provided herein are methods of sequencing regions of the genome that pose difficulties or challenges in sequencing using conventional sequencing methods, i.e. difficult to sequence polynucleotides. In some cases, difficult to sequence polynucleotides comprise low-complexity polynucleotides, repetitive polynucleotides, di-nucleotide repeat polynucleotides, tri-nucleotide repeat polynucleotides, GC-rich polynucleotides, polynucleotides with secondary structure, polynucleotides with 5'-YGN1-2AR motifs, and combinations thereof. In some cases, the difficult to sequence polynucleotide comprises a trinucleotide repeat, such as a CAG repeat, a CGG repeat, a GCC repeat, a GAA repeat, or a CTG repeat. In some cases, the difficult to sequence polynucleotide comprises a gene that is difficult to sequence such as an HLA gene, including an HLA-A gene, an HLA-B gene, an HLA-C gene, an HLA-E gene, an HLA-F gene, an HLA-G gene, an HLA-DP gene, an HLA-DQ gene, or an HLA-DR gene.

In some cases, obtaining the sequence of a gene that is difficult to sequence, such as an HLA gene, allows a healthcare provider to obtain a genotype of the patient at that genomic locus, for example an HLA genotype or an HLA-type. In some cases, an HLA-type is helpful in determining compatibility for an organ or tissue transplant, for example a bone marrow transplant, heart transplant, lung transplant, liver transplant, kidney transplant, pancreas transplant, intestine transplant, thymus transplant, cornea transplant, skin transplant, heart valve transplant, nerve transplant, or vein transplant.

Alternately, nucleic acid molecules that pose sequencing challenges include CYP2D6 genes and homologues thereof. Additionally, VDJ regions of immunoglobulin genes pose sequencing challenges.

### Methods herein are helpful in sequencing these genes and genomic regions

### Definitions

A partial list of relevant definitions is as follows.

"Amplified nucleic acid" or "amplified polynucleotide" includes any nucleic acid or polynucleotide molecule whose amount has been increased by any nucleic acid amplification or replication method performed *in vitro* as compared to its starting amount. For example, an amplified nucleic acid is optionally obtained from a polymerase chain reaction (PCR) which can, in some instances, amplify DNA in an exponential manner (for example, amplification to 2ⁿ copies in n cycles) wherein most products are generated from intermediate templates rather than directly from the sample template. Amplified nucleic acid is alternatively obtained from a linear amplification, where the amount increases linearly over time and which, in some cases, produces products that are synthesized directly from the sample.

"Amplification product" refers to a product resulting from an amplification reaction such as a polymerase chain reaction or a linear amplification.

An "amplicon" is a polynucleotide or nucleic acid that is the source and/or product of natural or artificial amplification or replication events.

The term "biological sample" or "sample" generally refers to a sample or part isolated from a biological entity. The biological sample, in some cases, shows the nature of the whole biological entity and examples include, without limitation, bodily fluids, dissociated tumor specimens, cultured cells, and any combination thereof. Biological samples come from one or more individuals. One or more biological samples come from the same individual. In one non limiting example, a first sample is obtained from an individual's blood and a second sample is obtained from an individual's tumor biopsy. Examples of biological samples include but are not limited to, blood, serum, plasma, nasal swab or nasopharyngeal wash, saliva, urine, gastric fluid, spinal fluid, tears, stool, mucus, sweat, earwax, oil, glandular secretion, cerebral spinal fluid, tissue, semen, vaginal fluid, interstitial fluids, including interstitial fluids derived from tumor tissue, ocular fluids, spinal fluid, throat swab, breath, hair, finger nails, skin, biopsy, placental fluid, amniotic fluid, cord blood, emphatic fluids, cavity fluids, sputum, pus, microbiota, meconium, breast milk and/or other excretions. In some cases, a blood sample comprises circulating tumor cells or cell free DNA, such as tumor DNA or fetal DNA. The samples include nasopharyngeal wash. Examples of tissue samples of the subject include but are not limited to, connective tissue, muscle tissue, nervous tissue, epithelial tissue, cartilage, cancerous or tumor sample, or bone. Samples are obtained from a human or an animal. Samples are obtained from a mammal, including vertebrates, such as murines, simians, humans, farm animals, sport animals, or pets. Samples are obtained from a living or dead subject. Samples are obtained fresh from a subject or have undergone some form of pre-processing, storage, or transport.

Nucleic acid sample as used herein refers to a nucleic acid sample for which sequence information is to be determined, A nucleic acid sample is extracted from a biological sample above, in some cases. Alternatively, a nucleic acid sample is artificially synthesized, synthetic, or de novo synthesized in some cases. The DNA sample is genomic in some cases, while in alternate cases the DNA sample is derived from a reverse-transcribed RNA sample.

"Bodily fluid" generally describes a fluid or secretion originating from the body of a subject. In some instances, bodily fluid is a mixture of more than one type of bodily fluid mixed together. Some non-limiting examples of bodily fluids include but are not limited to: blood, urine, bone marrow, spinal fluid, pleural fluid, lymphatic fluid, amniotic fluid, ascites, sputum, or a combination thereof.

"Complementary" or "complementarity," or, in some cases more accurately "reverse-complementarity" refer to nucleic acid molecules that are related by base-pairing. Complementary nucleotides are, generally, A and T (or A and U), or C and G (or G and U). Functionally, two single stranded RNA or DNA molecules are complementary when they form a double-stranded molecule through hydrogen-bond mediated base paring. Two single stranded RNA or DNA molecules are said to be substantially complementary when the nucleotides of one strand, optimally aligned and with appropriate nucleotide insertions or deletions, pair with at least about 90% to about 95% or greater complementarity, and more preferably from about 98% to about 100%) complementarity, and even more preferably with 100% complementarity. Alternatively, substantial complementarity exists when an RNA or DNA strand will hybridize under selective hybridization conditions to its complement. Selective hybridization conditions include, but are not limited to, stringent hybridization conditions and not stringent hybridization conditions. Hybridization temperatures are generally at least about 2° C to about 6° C lower than melting temperatures (Tₘ).

A "barcode" or "molecular barcode" includes a material for labeling. The barcode labels a molecule such as a nucleic acid or a polypeptide. The material for labeling is associated with information. A barcode in some instances is called a sequence identifier (for example, a sequence-based barcode or sequence index). In some cases, a barcode comprises a particular nucleotide sequence. A barcode is used as an identifier. A barcode is alternatively a different size molecule or different ending points of the same molecule. Barcodes include a specific sequence within the molecule and a different ending sequence. For example, a molecule that is amplified from the same primer and has 25 nucleotide positions is different than a molecule that is amplified and has 27 nucleotide positions. The additional positions in the 27mer sequence are optionally considered a barcode. A barcode is alternatively incorporated into a polynucleotide. A barcode is, in some cases, incorporated into a polynucleotide by many methods. Some non-limiting methods for incorporating a barcode include molecular biology methods. Some non-limiting examples of molecular biology methods to incorporate a barcode are through primers (*e.g.*, tailed primer elongation), probes (*i.e.*, elongation with ligation to a probe), or ligation (*i.e.*, ligation of known sequence to a molecule). In some cases, a barcode is biotinylated. A biotinylated barcode is optionally used as a means of purification. Purification of biotinylated molecules is accomplished through methods including but not limited to immunoprecipitation. In some cases, biotin is added at the target site and the sample DNA is sheared or otherwise cleaved, for example by endonucleases, yielding a sample sequence optionally at least 10 kilobases in length.

A barcode is variously incorporated into any region of a guide RNA or polynucleotide. In some cases, the region where the barcode is incorporated is known. Alternatively, the region is unknown. The barcode is contemplated to be added to any position along the guide RNA. In some cases barcode is added to the 5' end of a guide RNA. Alternatively, barcode is added to the 3' end of the guide RNA. In some cases, the barcode is added in between the 5' and 3' end of a guide RNA. A barcode is contemplated to be added with one or more other known sequences. One non-limiting example is the addition of a barcode with a sequence adapter.

Barcodes are contemplated to be associated with information. Some non-limiting examples of the type of information a barcode are associated with information include: the source of a sample; the orientation of a sample; the region or container a sample was processed in; the adjacent polynucleotide; or any combination thereof.

In some cases, barcodes are made from combinations of sequences (different from combinatorial barcoding) and are used to identify a sample or a genomic coordinate and a different template molecule or single strand the molecular label and copy of the strand was obtained from. In some cases a sample identifier, a genomic coordinate and a specific label for each biological molecule are amplified together. Barcodes, synthetic codes, or label information are obtained from the sequence context of the code (allowing for errors or error correcting), the length of the code, the orientation of the code, the position of the code within the molecule, and in combination with other natural or synthetic codes.

In some cases, incorporation of a barcode into a nucleic acid molecule indicates that the nucleic acid was present in a given sample at a given time period. In some cases, contiguous adjacent nucleic acid sequence sharing a common barcode or a common bar code pair is inferred to have been derived from a common molecule, particularly if the sample is diluted to less than an average of 2×, 1.5x, 1×, 0.7x, 0.5x, or 0.3x haploid genomes prior to barcode introduction.

Barcodes are contemplated herein to be added before pooling of samples. When the sequences of the pooled samples are determined, the barcode is sequenced along with the rest of the polynucleotide. The barcode is optionally used to associate the sequenced fragment with the source of the sample.

Barcodes are alternatively used to identify the strandedness sample. In some cases, one or more barcodes are used together. Two or more barcodes are alternatively adjacent to one another, not adjacent to one another, or any combination thereof. Adapter orientation is often used to determine strandedness. For example, if an "A" adapter is always in the 5'-3' direction in a first primer extension reaction, then one infers the read starting from the A adapter would be the compliment of the strand that was initially primed.

Barcodes are contemplated herein for use in combinatorial labeling.

As indicated herein, standard single-letter amino acid residue abbreviations as known in the art are used to refer to the twenty amino acids involved in cellular ribosomally driven polypeptide synthesis.

"Combinatorial labeling" is a method herein by which two or more barcodes are used to label a molecule. The two or more barcodes label a polynucleotide. The barcodes, each, alone in some cases are associated with information. Alternatively, the combination of the barcodes together is associated with information. In some cases a combination of barcodes is used together to determine in a randomly amplified molecule that the amplification occurred from the original sample template and not a synthetic copy of that template. In some cases, the length of one barcode in combination with the sequence of another barcode is used to label a polynucleotide. In some cases, the length of one barcode in combination with the orientation of another barcode is used to label a polynucleotide. In other cases, the sequence of one barcode is used with the orientation of another barcode to label a polynucleotide. In some cases the sequence of a first and a second bar code, in combination with the distance in nucleotides between them, is used to label or to identify a polynucleotide. In some cases the sequence of a first and a second bar code, in combination with the distance in nucleotides between them and the identity of the nucleotides between them, is used to label or to identify a polynucleotide.

"Degenerate" refers to a nucleic acid or nucleic acid region that is comprised of random bases. The terms "degenerate" and "random" are used interchangeably when referring to nucleic acid sequences (e.g., "degenerate primers" or "random primers" or "degenerate probes" or "random probes"). The degenerate region is of variable length. In some cases, the degenerate region comprises some portion of the whole nucleic acid (*e.g.*, a semi-degenerate primer). Alternatively, the degenerate region comprises the whole nucleic acid (*e.g.*, a "degenerate primer"). A degenerate nucleic acid mix, or semi-degenerate nucleic acid mix is comprised of every possible combination of base pairs, less than every possible combination of base pairs, or some combination of base pairs, a few combinations of base pairs, or a single base pair combination. A degenerate primer mix, or semi-degenerate primer mix comprises mixes of similar but not identical primers.

"Double-stranded" refers, in some cases, to two polynucleotide strands that have annealed through complementary base-pairing, such as in a reverse-complementary orientation.

"Known oligonucleotide sequence" or "known oligonucleotide" or "known sequence" refers to a polynucleotide sequence that is known. In some cases, a known oligonucleotide sequence corresponds to an oligonucleotide that has been designed, *e*.*g*., a universal primer for next generation sequencing platforms (*e*.*g*., Illumina, 454), a probe, an adaptor, a tag, a primer, a molecular barcode sequence, an identifier. A known sequence optionally comprises part of a primer. A known oligonucleotide sequence, in some cases, is not actually known by a particular user but is constructively known, for example, by being stored as data accessible by a computer. A known sequence is optionally a trade secret that is actually unknown or a secret to one or more users but is known by the entity who has designed a particular component of the experiment, kit, apparatus or software that the user is using.

"Library" in some cases refers to a collection of nucleic acids. A library optionally contains one or more target fragments. In some instances the target fragments comprise amplified nucleic acids. In other instances, the target fragments comprise nucleic acid that is not amplified. A library optionally contains nucleic acid that has one or more known oligonucleotide sequence(s) added to the 3' end, the 5' end or both the 3' and 5' end. The library is optionally prepared so that the fragments contain a known oligonucleotide sequence that identifies the source of the library (*e*.*g*., a molecular identification barcode identifying a patient or DNA source). In some instances, two or more libraries are pooled to create a library pool. Libraries are optionally generated with other kits and techniques such as transposon mediated labeling, or "tagmentation" as known in the art. Kits are commercially available. One non-limiting example of a kit is the Illumina NEXTERA kit (Illumina, San Diego, CA).

"Locus specific" or "loci specific" in some cases refers to one or more loci corresponding to a location in a nucleic acid molecule (*e*.*g*., a location within a chromosome or genome). In some instances, a locus is associated with genotype. In some instances loci are directly isolated and enriched from the sample, *e*.*g*., based on hybridization and/or other sequence-based techniques, or alternatively they may are selectively amplified using the sample as a template prior to detection of the sequence. In some instances, loci are selected on the basis of DNA level variation between individuals, based upon specificity for a particular chromosome, based on CG content and/or required amplification conditions of the selected loci, or other characteristics that will be apparent to one skilled in the art upon reading the present disclosure. A locus optionally refers to a specific genomic coordinate or location in a genome as denoted by the reference sequence of that genome.

"Long nucleic acid" refers, in some cases, to a polynucleotide longer than 1, 2, 3, 4, 5, 6, 7, 8, 9, or 10 kilobases.

The term "melting temperature" or "Tₘ" commonly refers to the temperature at which a population of double-stranded nucleic acid molecules becomes half dissociated into single strands. Equations for calculating the Tₘ of nucleic acids are well known in the art. One equation that gives a simple estimate of the Tₘ value is as follows: Tₘ=81.5+16.6(log 10[Na⁺])0.41(%[G+C])-675/n-1.0 m, when a nucleic acid is in aqueous solution having cation concentrations of 0.5 M or less, the (G+C) content is between 30% and 70%, n is the number of bases, and m is the percentage of base pair mismatches (see, *e*.*g*., Sambrook J et al., Molecular Cloning, A Laboratory Manual, 3rd Ed., Cold Spring Harbor Laboratory Press (2001)). Other references include more sophisticated computations, which take structural as well as sequence characteristics into account for the calculation of Tₘ.

"Nucleotide" refers to a base-sugar-phosphate combination. Nucleotides are monomeric units of a nucleic acid sequence (*e*.*g*., DNA and RNA). The term nucleotide includes naturally and non-naturally occurring ribonucleoside triphosphates ATP, TTP, UTP, CTG, GTP, and ITP, for example and deoxyribonucleoside triphosphates such as dATP, dCTP, dITP, dUTP, dGTP, dTTP, or derivatives thereof. Such derivatives include, for example, [aS]dATP, 7-deaza-dGTP and 7-deaza-dATP, and, for example, nucleotide derivatives that confer nuclease resistance on the nucleic acid molecule containing them. The term nucleotide as used herein also refers to dideoxyribonucleoside triphosphates (ddNTPs) and their derivatives. Illustrative examples of dideoxyribonucleoside triphosphates include, ddATP, ddCTP, ddGTP, ddITP, ddUTP and ddTTP, for example.

"Polymerase" refers to an enzyme that links individual nucleotides together into a strand, using another strand as a template.

"Polymerase chain reaction" or "PCR" refers to a technique for replicating a specific piece of selected DNA *in vitro*, even in the presence of excess non-specific DNA. Primers are added to the selected DNA, where the primers initiate the copying of the selected DNA using nucleotides and, typically, Taq polymerase or the like. By cycling the temperature, the selected DNA is repetitively denatured and copied. A single copy of the selected DNA, even if mixed in with other, random DNA, in some cases, is amplified to obtain thousands, millions, or billions of replicates. The polymerase chain reaction is used to detect and measure very small amounts of DNA and to create customized pieces of DNA.

The term "polynucleotides" or "nucleic acids" includes but is not limited to various DNA, RNA molecules, derivatives or combination thereof. These include species such as dNTPs, ddNTPs, DNA, RNA, peptide nucleic acids, cDNA, dsDNA, ssDNA, plasmid DNA, cosmid DNA, chromosomal DNA, genomic DNA, viral DNA, bacterial DNA, mtDNA (mitochondrial DNA), mRNA, rRNA, tRNA, nRNA, siRNA, snRNA, snoRNA, scaRNA, microRNA, dsRNA, ribozyme, riboswitch and viral RNA.

A "primer" generally refers to an oligonucleotide used to, *e.g.*, prime nucleotide extension, ligation and/or synthesis, such as in the synthesis step of the polymerase chain reaction or in the primer extension techniques used in certain sequencing reactions. A primer is alternatively used in hybridization techniques as a means to provide complementarity of a locus to a capture oligonucleotide for detection of a specific nucleic acid region.

"Primer extension product" refers to the product resulting from a primer extension reaction using a contiguous polynucleotide as a template, and a complementary or partially complementary primer to the contiguous sequence.

"Sequencing," "sequence determination," and the like generally refers to any and all biochemical methods that may be used to determine the order of nucleotide bases in a nucleic acid.

A "contig" refers to a nucleotide sequence that is assembled from two or more constituent nucleotide sequences that share common or overlapping regions of sequence homology. For example, the nucleotide sequences of two or more nucleic acid fragments are compared and aligned in order to identify common or overlapping sequences. Where common or overlapping sequences exist between two or more nucleic acid fragments, the sequences (and thus their corresponding nucleic acid fragments) are assembled into a single contiguous nucleotide sequence.

The term "biotin," as used herein, is intended to refer to biotin (5-[(3a*S*,4*S*,6a*R*)-2-oxohexahydro-1*H*-thieno[3,4-*d*]imidazol-4-yl]pentanoic acid) and any biotin derivatives and analogs. Such derivatives and analogs are substances which form a complex with the biotin binding pocket of native or modified streptavidin or avidin. Such compounds include, for example, iminobiotin, desthiobiotin and streptavidin affinity peptides, and also include biotin-.epsilon.-N-lysine, biocytin hydrazide, amino or sulfhydryl derivatives of 2-iminobiotin and biotinyl-ε-aminocaproic acid-N-hydroxysuccinimide ester, sulfo-succinimide-iminobiotin, biotinbromoacetylhydrazide, p-diazobenzoyl biocytin, 3-(N-maleimidopropionyl) biocytin. "Streptavidin" refers to a protein or peptide that binds to biotin and includes but is not limited to native egg-white avidin, recombinant avidin, deglycosylated forms of avidin, bacterial streptavidin, recombinant streptavidin, truncated streptavidin, and/or any derivative thereof.

A "subject" as used herein is a source of nucleic acid and in some cases refers to an organism that is currently living or an organism that at one time was living or an entity, optionally with a genome that replicates. The methods, kits, and/or compositions of the disclosure are contemplated herein to be applied to one or more single-celled or multi-cellular subjects, including but not limited to microorganisms such as bacterium and yeast; and animals including, but not limited to: humans; laboratory animals such as mice, rats, monkeys, and chimpanzees; domestic animals such as dogs and cats, and agricultural animals such as cows, horses, pigs, sheep, and goats. The methods of this disclosure are in some cases applied to germs or infectious agents, such as viruses or virus particles or one or more cells that have been infected by one or more viruses. In some cases, the subject is a fully synthetic organism.

A "support" is contemplated herein to be solid, semisolid, a bead, a surface. The support is optionally mobile in a solution or immobilized.

The term "unique identifier" includes but is not limited to a molecular bar code, or a percentage of a nucleic acid in a mix, such as dUTP.

"Repetitive sequence" as used herein refers to sequence that does not uniquely map to a single position in a nucleic acid sequence data set. Some repetitive sequence is optionally conceptualized as integer or fractional multiples of a repeating unit of a given size and exact or approximate sequence.

A "palindrome" or "palindromic sequence" as used herein refers to a nucleic acid sequence that is the same whether read 5' (five-prime) to 3' (three prime) on one strand or 5' to 3' on the complementary strand with which it forms a double helix.

An "inverted sequence" as used herein refers to a sequence that is the reverse sequence or reverse complement sequence relative to another sequence. A sequence is inverted if, upon (conceptually) rotating the molecule on which it is found by 180 degrees, the sequence as read in the same direction is the same sequence.

A "haplotype" as used herein refers to a collection of specific alleles in a cluster of tightly-linked genes on a chromosome that are likely to be inherited together.

A "sub-haplotype" as used herein refers to a subset of genes or portion of a haplotype.

The term "about" as used herein in reference to a number refers to that number plus or minus 10%.

The term "comprise" as used herein is inclusive, such that in the context of at least one element, it indicates that other unrecited elements may also be included.

As used herein, a repetitive or repeated region refers to a distinct genomic or other nucleic acid segment that recurs locally at a given locus or dispersed throughout a nucleic acid sample such as a genome sample. Exemplary repetitive segments include regions within the HLA locus, cyp2d6, VDJ regions and mobile elements such as Alu repeats and LINE elements.

Before the present methods, compositions and kits are described in greater detail, it is to be understood that this invention is not limited to particular method, composition or kit described, as such may, of course, vary. It is also to be understood that the terminology used herein is for the purpose of describing particular embodiments only, and is not intended to be limiting, since the scope of the present invention will be limited only by the appended claims as construed herein. Examples are put forth so as to provide those of ordinary skill in the art with a more complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is average molecular weight, temperature is in degrees Centigrade, and pressure is at or near atmospheric.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither or both limits are included in the smaller ranges is also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein are optionally used in the practice or testing of the present invention, some potential and preferred methods and materials are now described. All publications mentioned herein are incorporated herein by reference to disclose and describe the methods and/or materials in connection with which the publications are cited. It is understood that the present disclosure supersedes any disclosure of an incorporated publication to the extent there is a contradiction.

As will be apparent to those of skill in the art upon reading this disclosure, each of the individual embodiments described and illustrated herein has discrete components and features which may be readily separated from or combined with the features of any of the other several embodiments without departing from the scope or spirit of the present invention. Any recited method is contemplated to be carried out in the order of events recited or in any other order which is logically possible.

It must be noted that as used herein and in the appended claims, the singular forms "a", "an", and "the" include plural referents unless the context clearly dictates otherwise. Thus, for example, reference to "a cell" includes a plurality of such cells and reference to "the peptide" includes reference to one or more peptides and equivalents thereof, e.g. polypeptides, known to those skilled in the art, and so forth.

Turning to the figures, one sees the following:

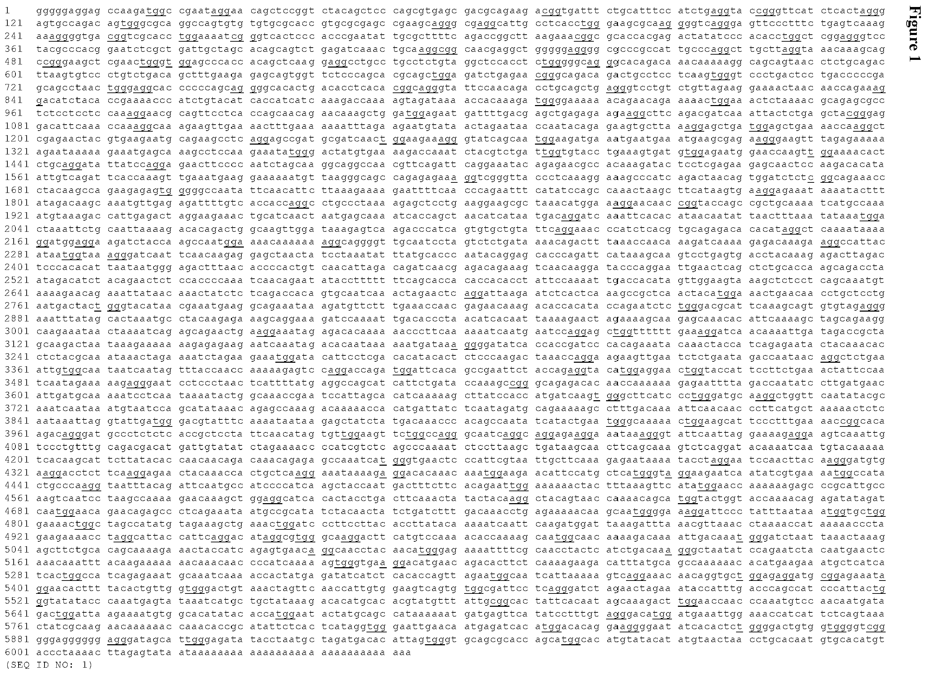

Figure 1 shows an exemplary human LINE1 (L1.4) repetitive element DNA sequence (SEQ ID NO: 1). PAM sequences 5'-3' in Figure 1 are underlined.

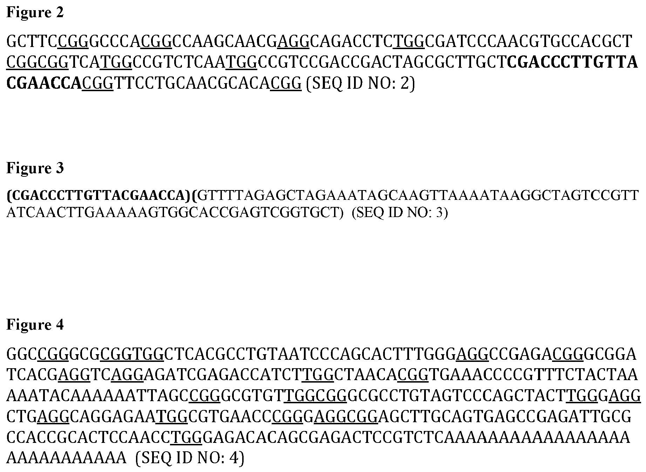

Figure 2 shows an exemplary consensus sequence of L1HA: Diagnostic sequence at 3' end of LINE-1 element (SEQ ID NO: 2). The PAM sequence is underlined. An example guide RNA complimentary sequence BOLD.
Figure 3 shows an exemplary guide RNA sequence (SEQ ID NO: 3).
Figure 4 shows an exemplary Alu-Y sequence (SEQ ID NO:4). Pam sequences are underlined in this figure.

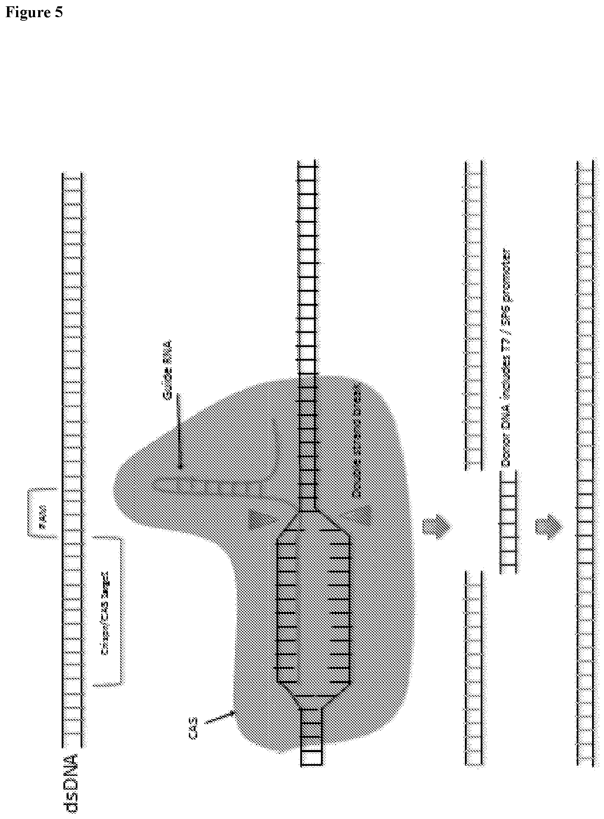

Figure 5 shows an exemplary CRISPR induced insertion of target specific T7 promoter sequences. High molecular weight double stranded genomic DNA is combined with CRISPR/CAS components including target specific guide RNA, CAS9 protein, donor DNA (including T7 promoter), and ligase. This figure shows that the T7 promoter is inserted specifically into target DNA sequence.

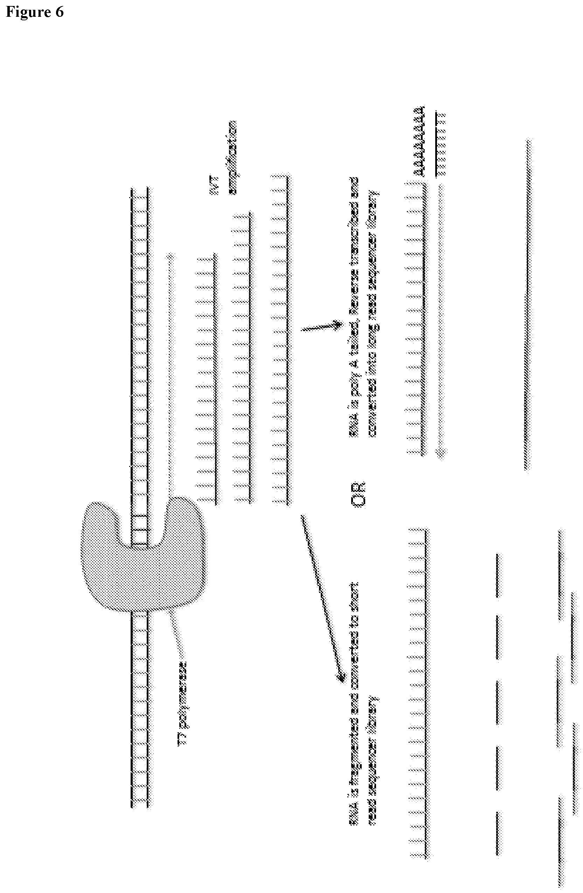

Figure 6 shows an exemplary in vitro transcription of target DNA generates amplified RNA copies of the target template. RNA molecules are optionally fragmented and converted into short read sequencer libraries or poly A tailed and reverse transcribed into full length cDNA. This figure illustrates that full length cDNA molecules are converted into long read sequencer libraries.

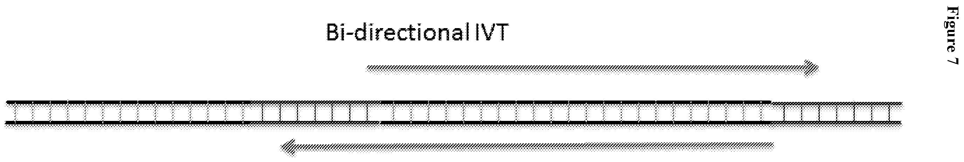

Figure 7 shows exemplary T7 promoters specifically inserted upstream and downstream of target sequence. In vitro transcription will occur in both directions covering target loci in excess of 10kb.

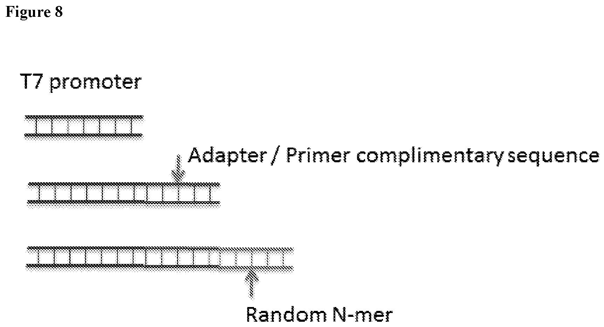

Figure 8 shows alternative donor DNA constructs. These constructs may comprise promoter sequences such as a T7 promoter, adaptor/primer complementary sequences, and random sequences.

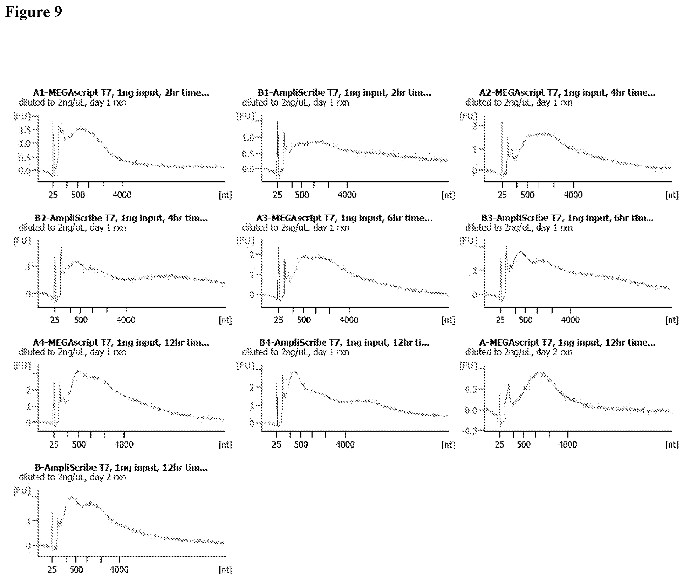

Figure 9 shows results of linear amplification of genomic sequence adjacent to an inserted T7 RNA polymerase site in a sample. Results are shown for two enzymes, run for 2, 4, 6, or 12 hours on 1ng of template diluted to 2ng/uL. For each result, the y-axis indicates fluorescence units, ranging from 0.0 to up to 1.0 or even in some cases 2.0. The x-axis represents library constituent length in nucleotides, on a logarithmic scale for which 25ntm 500nt and 4000nt are labeled. The results indicate that libraries having a median constituent size of between 500 bases and 2kb are routinely made through linear amplification directed by a RNA promoter inserted into a sample nucleic acid. In vitro transcription was performed for four separate time point intervals using MEGAscript and AmpliScribe T7 RNA polymerase kits. Reactions were run for 2, 4, 6, or 12 hour intervals. 1ng of DNA was used per reaction. Reactions were incubated with DNAse for 1 hour subsequent to transcription. RNA was quantified using a Qubit High Sensitivity RNA Assay kit. RNA analysis was performed using a High Sensitivity Pico mRNA Bioanalyzer.

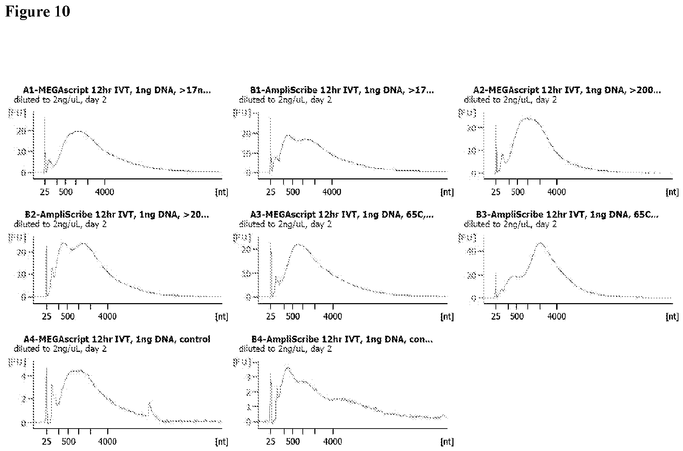

Turning to Figure 10, one sees the following. Bioanalyzer traces for size-selected RNA in vitro transcribed samples are presented. The x-axis indicates nucleotide length, [nt] on a logarithmic scale, with 25, 500 and 4000 nucleotides indicated. The y-axis indicates fluorescence units [FU]ranging from 0-20 in intervals of 10 (top and center file). 0-40 in intervals of 20 (center file, at right), or from 0-4 in intervals of 2 (bottom left) or 0-3 in intervals of 1 (bottom right). Results are presented for Ampliscribe and MEGAscript RNA polymerases. Samples were diluted to 2 ng/uL and run on day 2. The results indicate that in vitro transcribed samples are readily size selected to exclude smaller constituents.

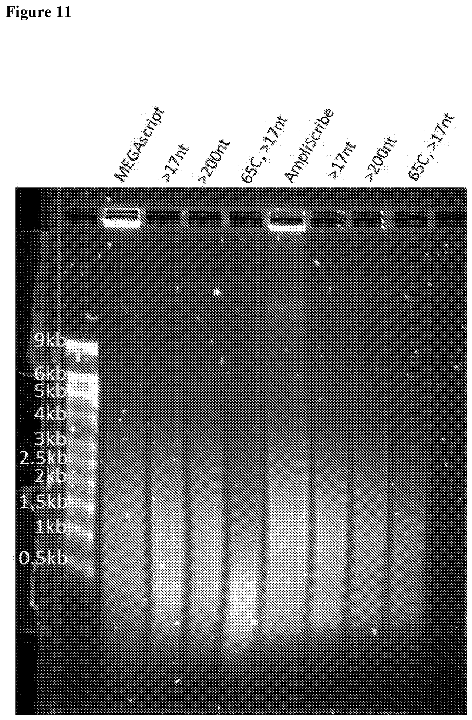

Turning to Figure 11, one sees the RNA of Figure 10 run on a denaturing agarose gel. The ladder at right indicates sizes of 0.5, 1, 1.5, 2, 2.5, 3, 4, 5, 6, and 9 kb. Sample lanes are, from left, MEGAscript control, MEGAscript 17nt exclusion, MEGAscript 200nt exclusion, MEGAscript 65C incubation plus 17nt exclusion, followed by Ampliscribe control, Ampliscribe 17nt exclusion, Ampliscribe 200nt exclusion, and Ampliscribe 65C incubation plus 17nt exclusion. The results indicate that in vitro transcribed samples are readily size selected to exclude smaller constituents.

### Partial list of numbered embodiments

The disclosure herein is further clarified in reference to a partial list of numbered embodiments as follows. 1. A method of determining a sequence adjacent to a region of known sequence of a nucleic acid molecule, the method comprising a) attaching a nucleic acid fragment comprising promoter sequence at the known region of the nucleic acid molecule; b) contacting the nucleic acid fragment to an RNA polymerase directed by the promoter; and c) synthesizing a plurality of RNA molecules; wherein a consensus sequence of the plurality of RNA molecules represents the sequence adjacent to the known region of a nucleic acid molecule. 2. The method of embodiment 1, wherein the consensus sequence is at least 10 kilobases in length. 3. The method of any one of embodiments 1 or 2, comprising treating the nucleic acid molecule using a DNase subsequent to synthesizing the plurality of RNA molecules. 4. The method of any one of embodiments 1 to 3, comprising reverse-transcribing the plurality of RNA molecules. 5. The method of any one of embodiments 1 to 4, comprising determining nucleic acid sequences of the plurality of RNA molecules. 6. The method of any one of embodiments 1 to 5, wherein the consensus sequence of the plurality of RNA molecules comprises sequence of molecules synthesized directly from the nucleic acid molecule. 7. The method of any one of embodiments 1 to 6, wherein the attaching comprises inserting the nucleic acid fragment comprising promoter sequence at the known region of the nucleic acid molecule. 8. The method of any one of embodiments 1 to 7, wherein the attaching comprises inserting the nucleic acid fragment comprising promoter sequence at the region of known sequence of the nucleic acid molecule. 9. The method of any one of embodiments 1 to 8, wherein the attaching comprises sequence-specific cleavage of the region of known sequence of the nucleic acid molecule. 10. The method of any one of embodiments 1 to 9, wherein the attaching comprises contacting the known region of the nucleic acid molecule to a CRISPR nucleic acid-protein complex. 11. The method of embodiment 10, wherein the CRISPR nucleic acid-protein complex comprises a guide RNA comprising SEQ ID NO: 3. 12. The method of any one of embodiments 1 to 11, wherein the attaching comprises ligating the nucleic acid fragment comprising promoter sequence. 13. The method of any one of embodiments 1 to 12, wherein the nucleic acid fragment comprising promoter sequence comprises a viral promoter. 14. The method of embodiment 13, wherein the viral promoter binds a viral RNA polymerase and is at least one promoter selected from the list consisting of T7, T3, T7lac, SP6, pL, CMV, SV40, and CaMV35S. 15. The method of any one of embodiments 1 to 12, wherein the nucleic acid fragment comprising promoter sequence comprises a bacterial promoter. 16. The method of embodiment 15, wherein the bacterial promoter binds a bacterial RNA polymerase and is at least one promoter selected from the list consisting of araBAD, trp, lac, and Ptac. 17. The method of any one of embodiments 1 to 12, wherein the nucleic acid fragment comprising promoter sequence comprises a eukaryotic promoter. 18. The method of embodiment 17, wherein the eukaryotic promoter binds a eukaryotic RNA polymerase and is at least one promoter selected from the list consisting of EF1a, PGK1, Ubc, beta actin, CAG, TRE, UAS, Ac5, Polyhedrin, CaMKIIa, ALB, GAL1, GAL10, TEF1, GDS, ADH1, Ubi, H1, and U6. 19. The method of embodiment 17, wherein the eukaryotic promoter is at least one promoter selected from the list consisting of an RNA pol I promoter, an RNA pol II promoter and an RNA pol III promoter. 20. The method of any one of embodiments 1 to 19, wherein the known region of a nucleic acid molecule comprises a repetitive element. 21. The method of embodiment 20, wherein the repetitive element comprises a mobile insertion element. 22. The method of embodiment 20, wherein the repetitive element comprises at least one of a LINE element, a SINE element, an Alu repeat, a transposon, a retrotransposon, a centromeric repeat, and a telomeric repeat. 23. The method of embodiment 20, wherein the LINE element comprises SEQ ID NO: 1. 24. A method of determining a plurality of locus-adjacent sequences of an element in a nucleic acid sample, comprising the steps of a) inserting a nucleic acid comprising a promoter into the element, b) generating a plurality of nucleic acid molecules directed by the promoter, and c) determining the sequence of the plurality of nucleic acid molecules, wherein the nucleic acid molecules are synthesized directly from the nucleic acid sample and wherein the plurality of nucleic acid molecules span locus adjacent sequences. 25. The method of embodiment 24, wherein the nucleic acid molecules comprise RNA. 26. The method of embodiment 24, wherein the nucleic acid molecules cannot prime nucleic acid synthesis. 27. The method of embodiment 24, wherein the nucleic acid sample comprises cancer cell nucleic acids. 28. The method of embodiment 24, wherein the nucleic acid sample comprises a single nuclear genome. 29. The method of embodiment 24, wherein the nucleic acid sample is obtained from a single cell. 30. The method of embodiment 24, comprising treating the nucleic acid sample using a DNase subsequent to synthesizing the plurality of RNA molecules. 31. The method of embodiment 24, comprising reverse-transcribing the plurality of RNA molecules. 32. The method of embodiment 24, wherein the plurality of nucleic acid molecules are RNA molecules. 33. The method of embodiment 24, wherein the consensus sequence of the plurality of RNA molecules comprises sequence of molecules synthesized directly from the nucleic acid molecule. 34. The method of embodiment 24, wherein the attaching comprises inserting the nucleic acid fragment comprising promoter sequence at the known region of the nucleic acid molecule. 35. The method of embodiment 24, wherein the attaching comprises inserting the nucleic acid fragment comprising promoter sequence at the known region of the nucleic acid molecule. 36. The method of embodiment 24, wherein the attaching comprises sequence-specific cleavage of the known region of the nucleic acid molecule. 37. The method of embodiment 24, wherein the attaching comprises contacting the known region of the nucleic acid molecule to a CRISPR nucleic acid-protein complex. 38. The method of embodiment 24, wherein the CRISPR nucleic acid-protein complex comprises a guide RNA comprising SEQ ID NO: 3. 39. The method of embodiment 24, wherein the attaching comprises ligating the nucleic acid fragment comprising promoter sequence. 40. The method of embodiment 24, wherein the nucleic acid fragment comprising promoter sequence comprises a viral promoter. 41. The method of embodiment 40, wherein the viral promoter is at least one promoter selected from the list consisting of T7, T3, T7lac, SP6, pL, CMV, SV40, and CaMV35S. 42. The method of embodiment 24, wherein the nucleic acid fragment comprising promoter sequence comprises a bacterial promoter. 43. The method of embodiment 42, wherein the bacterial promoter is at least one promoter selected from the list consisting of araBAD, trp, lac, and Ptac. 44. The method of embodiment 24, wherein the nucleic acid fragment comprising promoter sequence comprises a eukaryotic promoter. 45. The method of embodiment 44, wherein the eukaryotic promoter is at least one promoter selected from the list consisting of EF1a, PGK1, Ubc, beta actin, CAG, TRE, UAS, Ac5, Polyhedrin, CaMKIIa, ALB, GAL1, GAL10, TEF1, GDS, ADH1, Ubi, H1, and U6. 46. The method of embodiment 44, wherein the eukaryotic promoter is at least one promoter selected from the list consisting of an RNA pol I promoter, an RNA polII promoter and an RNA polIII promoter. 47. The method of embodiment 24, wherein the known region of a nucleic acid molecule comprises a repetitive element. 48. The method of embodiment 47, wherein the repetitive element comprises a mobile insertion element. 49. The method of embodiment 47, wherein the repetitive element comprises at least one of a LINE element, a SINE element, an Alu repeat, a transposon, a retrotransposon, a centromeric repeat, and a telomeric repeat. 50. The method of embodiment 47, wherein the LINE element comprises SEQ ID NO: 1. 51. A nucleic acid library comprising nucleic acids encoding border adjacent sequence for at least 90% of a repeated mobile element's borders in a nucleic acid sample. 52. The nucleic acid library of embodiment 51, wherein discrepancies between library constituents and the nucleic acid sample are independently derived. 53. The nucleic acid library of embodiment 51, wherein at least 50% of said repeated element's borders are present in at least 100 copies. 54. The nucleic acid library of embodiment 51, wherein library constituents are derived directly from the nucleic acid sample. 55. The nucleic acid library of embodiment 51, wherein library components are not clonally amplified prior to sequencing . 56. The nucleic acid library of embodiment 51, wherein the nucleic acid sample is derived from a single cell. 57. The nucleic acid library of embodiment 51, wherein the nucleic acid library is reverse transcribed from an RNA intermediate. 58. The nucleic acid library of embodiment 51, wherein the nucleic acid library comprises RNA. 59. The nucleic acid library of embodiment 51, wherein nucleic acid library constituents comprise promoter sequence. 60. The nucleic acid library of embodiment 59, wherein the RNA promoter sequence comprises at least one of a T7, T3, T7lac, SP6, pL, CMV, SV40, CaMV35S, araBAD, trp, lac, Ptac, EF1a, PGK1, Ubc, beta actin, CAG, TRE, UAS, Ac5, Polyhedrin, CaMKIIa, ALB, GAL1, GAL10, TEF1, GDS, ADH1, Ubi, H1, and U6. 61. The nucleic acid library of embodiment 51, wherein at least one border adjacent sequence indicates a defect in a gene related to at least one of cell cycle regulation, DNA repair, and growth regulation. 62. The nucleic acid library of embodiment 51, wherein nucleic acid library comprises nucleic acids encoding border adjacent sequence for at least 95% of a repeated mobile element's borders in a nucleic acid sample. 63. The nucleic acid library of embodiment 62, wherein nucleic acid library comprises nucleic acids encoding border adjacent sequence for at least 99% of a repeated mobile element's borders in a nucleic acid sample. 64. The nucleic acid library of embodiment 51, wherein at least 50% of the library constituent nucleic acids are located on a nucleic acid within 20kb of a mobile element border. 65. The nucleic acid library of embodiment 51, wherein at least 75% of the library constituent nucleic acids are located on a nucleic acid within 20kb of a mobile element border in proximity to a mobile element border. 66. The nucleic acid library of embodiment 51, wherein at least 90% of the library constituent nucleic acids are located on a nucleic acid within 20kb of a mobile element border. 67. The nucleic acid library of embodiment 51, wherein at least 50% of the library constituent nucleic acids are located on a nucleic acid within 10kb of a mobile element border. 68. The nucleic acid library of embodiment 51, wherein at least 75% of the library constituent nucleic acids are located on a nucleic acid within 10kb of a mobile element border in proximity to a mobile element border. 69. The nucleic acid library of embodiment 51, wherein at least 90% of the library constituent nucleic acids are located on a nucleic acid within 10kb of a mobile element border. 70. The nucleic acid library of embodiment 51, wherein at least 50% of the library constituent nucleic acids are located on a nucleic acid within 5kb of a mobile element border. 71. The nucleic acid library of embodiment 51, wherein at least 75% of the library constituent nucleic acids are located on a nucleic acid within 5kb of a mobile element border in proximity to a mobile element border. 72. The nucleic acid library of embodiment 51, wherein at least 90% of the library constituent nucleic acids are located on a nucleic acid within 5kb of a mobile element border. 73. The nucleic acid library of embodiment 51, wherein at least 50% of the library constituent nucleic acids are located on a nucleic acid within 1kb of a mobile element border. 74. The nucleic acid library of embodiment 51, wherein at least 75% of the library constituent nucleic acids are located on a nucleic acid within 1kb of a mobile element border in proximity to a mobile element border. 75. The nucleic acid library of embodiment 51, wherein at least 90% of the library constituent nucleic acids are located on a nucleic acid within 1kb of a mobile element border. 76. The nucleic acid library of any one of embodiments 51 to 75, wherein the mean fragment length is about 500 bases. 77. The nucleic acid library of any one of embodiments 51 to 75, wherein the mean fragment length is about 1000 bases. 78. The nucleic acid library of any one of embodiments 51 to 75, wherein the median fragment length is about 500 bases. 79. The nucleic acid library of any one of embodiments 51 to 75, wherein the median fragment length is about 1000 bases. 80. A composition comprising a targeting sequence and a promoter, wherein the targeting sequence comprises a nucleic acid sequence that directs insertion of the composition into one or more specific locations in a nucleic acid sequence and the promoter comprises a nucleic acid sequence that directs synthesis of a nucleic acid from a sample sequence adjacent to the insertion of the promoter. 81. The composition of embodiment 80, wherein the targeting sequence comprises a nucleic acid sequence homologous to the specific location. 82. The composition of embodiment 80, wherein the targeting sequence comprises a nucleic acid sequence that base pairs to the specific location. 83. The composition of embodiment 80, wherein the targeting sequence comprises a nucleic acid sequence that hybridizes to the specific location. 84. The composition of any one of embodiments 80 to 83, wherein the targeting sequence comprises at least one of clustered regularly interspaced short palindromic repeats (CRISPR) sequence, a zinc finger nuclease (ZFN) sequence, and a transcription activator-like effector nucleases (TALENs) sequence. 85. The composition of embodiment 84, wherein the CRISPR sequence comprises a guide RNA with a sequence comprising SEQ ID NO: 3. 86. The composition of any one of embodiments 80 to 85, wherein the promoter comprises at least one of a bacterial promoter, a viral promoter, and a eukaryotic promoter. 87. The composition of embodiment 86, wherein the bacterial promoter comprises at least one of araBAD, trp, lac, and Ptac. 88. The composition of embodiment 86, wherein the viral promoter comprises at least one of T7, T7lac, SP6, pL, CMV, SV40, and CaMV35S. 89. The composition of embodiment 86, wherein the eukaryotic promoter comprises at least one of EF1a, PGK1, Ubc, beta actin, CAG, TRE, UAS, Ac5, Polyhedrin, CaMKIIa, ALB, GAL1, GAL10, TEF1, GDS, ADH1, Ubi, H1, and U6. 90. The composition of any one of embodiments 80 to 89, wherein the specific location in the nucleic acid sequence comprises a low-complexity nucleic acid sequence. 91. The composition of any one of embodiments 80 to 90, wherein the specific location in the nucleic acid sequence comprises a repetitive nucleic acid sequence. 92. The composition of any one of embodiments 80 to 91, wherein the low-complexity nucleic acid sequence or the repetitive nucleic acid sequence comprises at least one of a tri-nucleotide repeat, tandem repeat, and human leukocyte antigen gene. 93. The composition of any one of embodiments 80 to 91, wherein the specific location in the nucleic acid sequence comprises a mobile genetic element. 94. The composition of embodiment 93, wherein the mobile genetic element comprises at least one of a transposon, a retrotransposon, a DNA transposon, an insertion sequence, a plasmid, a bacteriophage, a group II intron, a group I intron, an Alu element, a MIR element, an intracisternal A particle (IAP), an ETn, a virus, and a fragments thereof. 95. The composition of embodiment 94, wherein the retrotransposon comprises at least one of a transposable element, a LINE, a SINE, and fragments thereof. 96. The composition of embodiment 94, wherein the LINE comprises SEQ ID NO: 1. 97. The composition of embodiment 94, wherein the virus comprises a retrovirus or a fragment thereof. 98. The composition of any one of embodiments 80 to 97, wherein the nucleic acid synthesis comprises at least one of RNA transcription and DNA synthesis. 99. A method of determining a nucleic acid sequence adjacent to a nucleic acid sequence of interest comprising: (a) inserting a targeting nucleic acid sequence comprising a targeting sequence and a promoter into one or more specific locations in the nucleic acid sequence of interest, (b) directing synthesis of a nucleic acid from the promoter, and (c) sequencing the synthesized nucleic acid. 100. The method of embodiment 99, wherein the targeting sequence comprises at least one of a clustered regularly interspaced short palindromic repeats (CRISPR) sequence, a zinc finger nuclease (ZFN) sequence, and a transcription activator-like effector nucleases (TALENs) sequence. 101. The composition of embodiment 100, wherein the CRISPR sequence comprises a guide RNA with a sequence comprising SEQ ID NO: 3. 102. The method of embodiment any one of embodiments 99 to 101, wherein the promoter comprises at least one of a bacterial promoter, a viral promoter, and a eukaryotic promoter. 103. The method of embodiment 102, wherein the bacterial promoter comprises at least one of araBAD, trp, lac, and Ptac. 104. The method of embodiment 102, wherein the viral promoter comprises at least one of T7, T7lac, SP6, pL, CMV, SV40, and CaMV35S. 105. The method of embodiment 102, wherein eukaryotic promoter comprises at least one of EF1a, PGK1, Ubc, beta actin, CAG, TRE, UAS, Ac5, Polyhedrin, CaMKIIa, GAL1, GAL10, TEF1, GDS, ADH1, Ubi, H1, and U6. 106. The method of any one of embodiments 99 to 105, wherein the sequence of interest comprises a low-complexity nucleic acid sequence. 107. The method of any one of embodiments 99 to 106, wherein the sequence of interest comprises a repetitive nucleic acid sequence. 108. The method of any one of embodiments 99 to 107, wherein the sequence of interest comprises at least one of tri-nucleotide repeat, tandem repeat, and human leukocyte antigen gene. 109. The method of any one of embodiments 99 to 108, wherein the sequence of interest comprises a mobile genetic element. 110. The method of embodiment 109, wherein the mobile genetic element comprises a transposon, a retrotransposon, a DNA transposon, an insertion sequence, a plasmid, a bacteriophage, a group II intron, a group I intron, an Alu element, a MIR element, an intracisternal A particle (IAP), an ETn, a virus, or a fragment thereof. 111. The method of embodiment 110, wherein the retrotransposon comprises at least one of transposable element, a LINE, a SINE, and fragments thereof. 112. The method of embodiment 111, wherein the LINE comprises SEQ ID NO: 1. 113. The method of embodiment 110, wherein the virus comprises at least one of a retrovirus and fragments thereof. 114. The method of any one of embodiments 99 to 113, wherein nucleic acid synthesis comprises at least one of RNA transcription and DNA synthesis. 115. The method of embodiment 114, wherein RNA transcription comprises use of a RNA polymerase. 116. The method of embodiment 115, wherein the RNA polymerase comprises at least one of a T7 RNA polymerase, a T3 RNA polymerase, a SP6 RNA polymerase, a RNA polymerase I, a RNA polymerase II, a RNA polymerase III, a RNA polymerase IV, a RNA polymerase V, and a single subunit RNA polymerase. 117. The method of any one of embodiments 99 to 114, wherein DNA synthesis comprises use of a DNA polymerase. 118. The method of embodiment 117, wherein the DNA polymerase comprises at least one of a T7 DNA polymerase, a T3 DNA polymerase, a SP6 DNA polymerase, a DNA polymerase I, a DNA polymerase II, a DNA polymerase III, a Taq DNA polymerase, and a Pfu DNA polymerase. 119. The method of any one of embodiments 99 to 118, wherein the nucleic acid synthesis requires a primer. 120. The method of any one of embodiments 99 to 119, wherein the synthesized nucleic acid is synthesized directly from the nucleic acid sequence of interest. 121. The method of any one of embodiments 99 to 120, wherein the nucleic acid is synthesized without introducing a mutation. 122. The method of embodiment 121, wherein the mutation is at least one of a point mutation, a deletion, an insertion, and a chimera. 123. The method of any one of embodiments 99 to 122, wherein the synthesized nucleic acid comprises DNA. 124. The method of any one of embodiments 99 to 122, wherein the synthesized nucleic acid comprises cDNA. 125. The method of embodiment 123 or embodiment 124, wherein the synthesized nucleic acid is treated with an RNase. 126. The method of any one of embodiments 99 to 122, wherein the synthesized nucleic acid is a RNA. 127. The method of embodiment 126, wherein the synthesized nucleic acid is treated with a DNase. 128. The method of any one of embodiments 99 to 127, wherein the sequencing comprises at least one of Sanger sequencing, Next-generation sequencing, pyrosequencing, Massively parallel signature sequencing, single molecule real-time sequencing, ion torrent sequencing, sequencing by synthesis, and sequencing by ligation. 129. The method of any one of embodiments 99 to 128, wherein the method detects a mutation in a subject. 130. The method of any one of embodiments 99 to 128, wherein the method detects a mutation in a tissue sample obtained from a subject. 131. The method of embodiment 130, wherein the tissue sample comprises at least one of tumor, blood, saliva, sputum, skin, and epithelial tissue. 132. A method of mapping a site of insertion of a DNA element in a nucleic acid sample from a subject, comprising: i) inserting a targeting nucleic acid sequence comprising a targeting sequence and a promoter by contacting the genomic DNA with the targeting sequence and one or more reagents sufficient to insert the targeting sequence into the DNA element; ii) creating an amplified nucleic acid directly from the genomic DNA by contacting the inserted targeting sequence with one or more enzymes that catalyze nucleic acid synthesis from the promoter thereby creating an amplified nucleic acid; iii) sequencing the amplified nucleic acid. 133. The method of embodiment 132, wherein the targeting sequence comprises at least one of a clustered regularly interspaced short palindromic repeats (CRISPR) sequence, a zinc finger nuclease (ZFN) sequence, and a transcription activator-like effector nucleases (TALENs) sequence. 134. The composition of embodiment 133, wherein the CRISPR sequence comprises a guide RNA with a sequence comprising SEQ ID NO: 3. 135. The method of any one of embodiments 132 to 134, wherein the promoter comprises at least one of a bacterial promoter, a viral promoter, and a eukaryotic promoter. 136. The method of embodiment 135, wherein the bacterial promoter comprises at least one of araBAD, trp, lac, and Ptac. 137. The method of embodiment 135, wherein the viral promoter comprises at least one of T7, T7lac, SP6, pL, CMV, SV40, and CaMV35S. 138. The method of embodiment 135, wherein eukaryotic promoter comprises at least one of EF1a, PGK1, Ubc, beta actin, CAG, TRE, UAS, Ac5, Polyhedrin, CaMKIIa, GAL1, GAL10, TEF1, GDS, ADH1, Ubi, H1, and U6. 139. The method of any one of embodiments 132 to 138, wherein the DNA element comprises a low-complexity nucleic acid sequence. 140. The method of any one of embodiments 132 to 139, wherein the DNA element comprises a repetitive nucleic acid sequence. 141. The method of any one of embodiments 132 to 140, wherein the DNA element comprises at least one of a tri-nucleotide repeat, and tandem repeat. 142. The method of any one of embodiments 132 to 141, wherein the DNA element comprises a mobile genetic element. 143. The method of embodiment 142, wherein the mobile genetic element comprises at least one of a transposon, a retrotransposon, a DNA transposon, an insertion sequence, a plasmid, a bacteriophage, a group II intron, a group I intron, an Alu element, a MIR element, an intracisternal A particle (IAP), an ETn, a virus, and fragments thereof. 144. The method of embodiment 143, wherein the retrotransposon comprises at least one of a transposable element, a LINE, a SINE, and fragments thereof. 145. The method of embodiment 144, wherein the LINE comprises SEQ ID NO: 1. 146. The method of embodiment 143, wherein the virus comprises a retrovirus or a fragment thereof. 147. The method of embodiment 132 to 146, wherein the enzyme comprises a RNA polymerase. 148. The method of embodiment 147, wherein the RNA polymerase comprises at least one of a T7 RNA polymerase, a T3 RNA polymerase, a SP6 RNA polymerase, a RNA polymerase I, a RNA polymerase II, a RNA polymerase III, a RNA polymerase IV, a RNA polymerase V, and a single subunit RNA polymerase. 149. The method of any one of embodiments 132 to 148, wherein the enzyme a DNA polymerase. 150. The method of embodiment 149, wherein the DNA polymerase comprises at least one of a T7 DNA polymerase, a T3 DNA polymerase, a SP6 DNA polymerase, a DNA polymerase I, a DNA polymerase II, a DNA polymerase III, a Taq DNA polymerase, and a Pfu DNA polymerase. 151. The method of any one of embodiments 132 to 150, wherein the nucleic acid synthesis requires a primer. 152. The method of any one of embodiments 132 to 151, wherein the synthesized nucleic acid is synthesized directly from the nucleic acid sequence of interest. 153. The method of any one of embodiments 132 to 152, wherein the nucleic acid is synthesized without introducing a mutation. 154. The method of embodiment 153, wherein the mutation is at least one of a point mutation, a deletion, an insertion, and a chimera. 155. The method of any one of embodiments 132 to 154, wherein the synthesized nucleic acid is a DNA. 156. The method of any one of embodiments 132 to 154, wherein the synthesized nucleic acid is a cDNA. 157. The method of embodiment 155 or embodiment 156, wherein the synthesized nucleic acid is treated with an RNase. 158. The method of any one of embodiments 132 to 154, wherein the synthesized nucleic acid is a RNA. 159. The method of embodiment 158, wherein the synthesized nucleic acid is treated with a DNase. 160. The method of any one of embodiments 132 to 159, wherein the sequencing comprises at least one of Sanger sequencing, Next-generation sequencing, pyrosequencing, Massively parallel signature sequencing, single molecule real-time sequencing, ion torrent sequencing, sequencing by synthesis, and sequencing by ligation. 161. The method of any one of embodiments 132 to 160, wherein the method detects a mutation in a subject. 162. The method of any one of embodiments 132 to 160, wherein the method detects a mutation in a tissue sample obtained from a subject. 163. The method of embodiment 162, wherein the tissue sample comprises at least one of tumor, blood, saliva, sputum, skin, and epithelial tissue. 164. A method of sequencing a repetitive genomic region comprising: i) inserting a targeting nucleic acid sequence comprising a targeting sequence and a promoter by contacting the genomic DNA with the targeting sequence and one or more reagents sufficient to insert the targeting sequence into the repetitive genomic region; ii) creating an amplified nucleic acid directly from the genomic DNA by contacting the inserted targeting sequence with one or more enzymes that catalyze nucleic acid synthesis from the promoter thereby creating an amplified nucleic acid; iii) sequencing the amplified nucleic acid. 165. The method of embodiment 164, wherein the targeting sequence comprises at least one of a clustered regularly interspaced short palindromic repeats (CRISPR) sequence, a zinc finger nuclease (ZFN) sequence, and a transcription activator-like effector nucleases (TALENs) sequence. 166. The composition of embodiment 165, wherein the CRISPR sequence comprises a guide RNA with a sequence comprising SEQ ID NO: 3. 167. The method of any one of embodiments 164 to 166, wherein the promoter comprises at least one of a bacterial promoter, a viral promoter, and a eukaryotic promoter. 168. The method of embodiment 167, wherein the bacterial promoter comprises at least one of araBAD, trp, lac, and Ptac. 169. The method of embodiment 167, wherein the viral promoter comprises at least one of T7, T7lac, SP6, pL, CMV, SV40, and CaMV35S. 170. The method of embodiment 167, wherein eukaryotic promoter comprises at least one of EF1a, PGK1, Ubc, beta actin, CAG, TRE, UAS, Ac5, Polyhedrin, CaMKIIa, GAL1, GAL10, TEF1, GDS, ADH1, Ubi, H1, and U6. 171. The method of any one of embodiments 164 to 170, wherein the repetitive genomic region comprises a low-complexity nucleic acid sequence. 172. The method of any one of embodiments 164 to 171, wherein the repetitive genomic region comprises a repetitive nucleic acid sequence. 173. The method of any one of embodiments 164 to 172, wherein the repetitive genomic region comprises at least one of a tri-nucleotide repeat and tandem repeat. 174. The method of any one of embodiments 164 to 173, wherein the repetitive genomic region comprises a mobile genetic element. 175. The method of embodiment 174, wherein the mobile genetic element comprises at least one of a transposon, a retrotransposon, a DNA transposon, an insertion sequence, a plasmid, a bacteriophage, a group II intron, a group I intron, an Alu element, a MIR element, an intracisternal A particle (IAP), an ETn, a virus, and fragments thereof. 176. The method of embodiment 175, wherein the retrotransposon comprises at least one of a transposable element, a LINE, a SINE, and fragments thereof. 177. The method of embodiment 176, wherein the LINE comprises SEQ ID NO: 1. 178. The method of embodiment 175, wherein the virus comprises a retrovirus or a fragment thereof. 179. The method of embodiment 164 to 178, wherein the enzyme comprises a RNA polymerase. 180. The method of embodiment 179, wherein the RNA polymerase comprises at least one of a T7 RNA polymerase, a T3 RNA polymerase, a SP6 RNA polymerase, a RNA polymerase I, a RNA polymerase II, a RNA polymerase III, a RNA polymerase IV, a RNA polymerase V, and a single subunit RNA polymerase. 181. The method of any one of embodiments 164 to 178, wherein the enzyme comprises a DNA polymerase. 182. The method of embodiment 181, wherein the DNA polymerase comprises at least one of a T7 DNA polymerase, a T3 DNA polymerase, a SP6 DNA polymerase, a DNA polymerase I, a DNA polymerase II, a DNA polymerase III, a Taq DNA polymerase, and a Pfu DNA polymerase. 183. The method of any one of embodiments 164 to 182, wherein the nucleic acid synthesis requires a primer. 184. The method of any one of embodiments 164 to 183, wherein the synthesized nucleic acid is synthesized directly from the nucleic acid sequence of interest. 185. The method of any one of embodiments 164 to 184, wherein the nucleic acid is synthesized without introducing a mutation. 186. The method of embodiment 185, wherein the mutation is at least one of a point mutation, a deletion, an insertion, and a chimera. 187. The method of any one of embodiments 164 to 186, wherein the synthesized nucleic acid is a DNA. 188. The method of any one of embodiments 164 to 186, wherein the synthesized nucleic acid is a cDNA. 189. The method of embodiment 187 or embodiment 188, wherein the synthesized nucleic acid is treated with an RNase. 190. The method of any one of embodiments 164 to 186, wherein the synthesized nucleic acid is a RNA. 191. The method of embodiment 190, wherein the synthesized nucleic acid is treated with a DNase. 192. The method of any one of embodiments 164 to 191, wherein the sequencing comprises at least one of Sanger sequencing, Next-generation sequencing, pyrosequencing, Massively parallel signature sequencing, single molecule real-time sequencing, ion torrent sequencing, sequencing by synthesis, and sequencing by ligation. 193. The method of any one of embodiments 164 to 192, wherein the method detects a mutation in a subject. 194. The method of any one of embodiments 164 to 192, wherein the method detects a mutation in a tissue sample obtained from a subject. 195. The method of embodiment 194, wherein the tissue sample comprises at least one of tumor, blood, saliva, sputum, skin, and epithelial tissue.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

### EXAMPLES

The following examples are given for the purpose of illustrating various embodiments of the invention and are not meant to limit the present invention in any fashion. The present examples, along with the methods described herein are presently representative of preferred embodiments, are exemplary, and are not intended as limitations on the scope of the invention. Changes therein and other uses which are encompassed within the spirit of the invention as defined by the scope of the claims will occur to those skilled in the art.

### Example 1: Target specific amplification of HLA genes.

HLA regions are notoriously hard to assemble with short read sequencers. They are both highly polymorphic and highly repetitive. Guide RNA designed upstream of the HLA-A gene promoter in example coordinates chr6:29,940,000-29,942,000 will have the potential to generate T7 transcribed RNA products spanning the entire HLA gene at about 5kb of sequence.

In order to sequence the HLA gene, a plasmid is designed to include the targeted sequence from the HLA promoter with a T7 promoter inserted. The targeted sequence is selected to work most effectively with Cas9 in the CRISPR system. Once the plasmid containing the target sequence and the T7 promoter is designed and prepared, a guide RNA is transcribed and resulting guide RNA is incubated with the genomic DNA sample and isolated Cas9 enzyme. Alternatively, the guide RNA is synthesized or generated from a double stranded linear template. The resulting product, a tagged genomic DNA sample that has a T7 promoter inserted in the promoter region of the HLA-A gene, is then purified. The tagged genomic DNA sample is incubated with nucleotides and a T7 RNA polymerase which creates an RNA transcript from the HLA-A gene. Each transcript is made directly from the tagged genomic DNA sample and no errors such as insertions, deletions, or point mutations are made. The RNA sample is then purified from the tagged genomic DNA sample and polyadenylated. A cDNA is made from the RNA sample in a reverse transcriptase reaction and oligo-dT. The RNA is removed from the cDNA using RNase H and the cDNA is used in sequencing reactions to determine the sequence of the HLA-A gene with high accuracy possible in this method. The sequence of the HLA-A gene provides information about the subject from whom the genomic DNA sample was taken and thereby provides an accurate HLA typing at the HLA-A gene.

### Example 2: Identification of somatic mobile elements such as LINE-1 rearrangements in tumors.

LINE -1 rearrangements are estimated to rearrange genomic material once in every 20 cell divisions. The mechanism behind transposition may include the copy and paste of genomic DNA sequence outside of the canonical LINE-1 element sequence and insert that sequence in a new position. Published examples of this "tag along" genomic material is in some cases as long as 10 kilobases. Short read sequences do not have the ability to map these events as mapping based assembly will not position short reads corresponding to shuffled genomic material in a new location that is in conflict with the reference genome used for short read assembly. The ability to sequence through a contiguous molecule of lengths greater than 10 kilobases and into the flanking genomic sequence has the ability to identify and quantify these events. A guide RNA with complimentary target sequence to conserved regions of the human LINE-1 element enables T7 based transcription from the conserved LINE-1 diagnostic sequence out toward the flanking sequence. A comparison of tumor and normal sequencing of the products reveals somatic LINE-1 rearrangements with unparalleled accuracy. Multiple T7 insertions along both 3' and 5' ends as well as throughout the conserved LINE-1 element sequence adds the ability to identify full length somatic L1 transposition in tumors. It is estimated that 60% of tumors have somatic L1 events.

In order to map the sites of LINE-1 rearrangements in tumors, a plasmid is constructed to contain a targeted sequence complementary to a sequence in the LINE-1 element and a T7 promoter. The targeted sequence is selected to work most effectively with Cas9 in the CRISPR system. A guide RNA is made from the plasmid, the guide RNA containing the targeted sequence and the T7 promoter. The guide RNA is incubated with the genomic DNA sample from the tumor and isolated Cas9 enzyme. The resulting product, a tagged genomic DNA sample that has a T7 promoter inserted into the targeted LINE-1 sequence, is purified. The tagged genomic DNA sample is incubated with nucleotides and a T7 RNA polymerase which creates an RNA transcript from the LINE-1 element. Each transcript is made directly from the tagged genomic DNA sample and no errors such as insertions deletions, or point mutations are made. The RNA sample is then purified from the tagged genomic DNA sample and polyadenylated. A cDNA is made from the RNA sample in a reverse transcriptase reaction and oligo-dT. The RNA is removed from the cDNA using RNaseH and the cDNA is used in sequencing reactions to determine the sequence of the genomic DNA adjacent to the LINE-1 element and thereby the location of the LINE-1 element. The location of the element and any additional LINE-1 elements in the tumor sample gives diagnostic information to the physician such as specific treatments that may work to cure the tumor.

### Example 3: Determining length of tri-nucleotide repeat length in Huntington's disease

Huntington's disease is a neurodegenerative genetic disorder that affects muscle coordination, cognitive ability, and behavior. A well-documented mutation in the Huntingtin gene is responsible for the disease, which is inherited in an autosomal dominant fashion. The mutation is an expansion, from one generation in a family to the next, of a CAG trinucleotide repeat stretch found in the coding sequence of the gene. This CAG trinucleotide encodes the amino acid glutamine, so expansion of the CAG repeat results in expansion of a polyglutamine stretch in the resulting protein. Obtaining an exact sequence of the expanded polynucleotide region presents challenges. As the size of the repeat region affects the disease status of the patient it is desirable to determine the sequence and therefore the size of the repeat region.

In order to determine the size of the CAG repeat, a plasmid is constructed to contain a targeted sequence complementary to a sequence in the Huntingtin gene and a T7 promoter. The targeted sequence is selected to work most effectively with Cas9 in the CRISPR system. A guide RNA is made from the plasmid, the guide RNA containing the targeted sequence and the T7 promoter. The guide RNA is incubated with the genomic DNA sample from the tumor and isolated Cas9 enzyme. The resulting product, a tagged genomic DNA sample that has a T7 promoter inserted into the targeted Huntingtin sequence, is purified. The tagged genomic DNA sample is incubated with nucleotides and a T7 RNA polymerase which creates an RNA transcript from the Huntingtin gene. Each transcript is made directly from the tagged genomic DNA sample and no errors such as insertions deletions, or point mutations are made. The RNA sample is then purified from the tagged genomic DNA sample and polyadenylated. A cDNA is made from the RNA sample in a reverse transcriptase reaction and oligo-dT. The RNA is removed from the cDNA using RNaseH and the cDNA is used in sequencing reactions to determine the sequence of the CAG repeat in the Huntingtin gene. The number of CAG repeats in the Huntingtin gene gives diagnostic information to the physician and the patient regarding the expected severity of disease.

### Example 4: Using CRISPR/CAS to insert hairpin tags into the genome

A Cyp2d6 gene is selected for sequencing using CRISPR/CAS to create a double stranded break at a target site in the genome at the genomic locus of the gene. The double stranded break is made into a sticky end by treating the DNA sample with an exonuclease exposing one strand of the target site. The tagged nucleic acid has a portion with a nucleic acid sequence complementary to the exposed strand, a T7 promoter, and a portion that is self-complementary and forms a hairpin. DNA ligase ligates the tagged nucleic acid to the target site thereby incorporating a T7 promoter near the Cyp2d6 gene. The hairpin tag is efficient at ligating the tag to the target site and the site is ready for targeted RNA transcription of the Cyp2d6 gene. The tag allows the Cyp2d6 gene to be uniquely tagged for sequencing, differentiating from the Cyp2d6 pseudogene.

### Example 5: Size selection of in vitro transcribed RNA

In vitro transcription was performed on 1 ng DNA samples comprising T7 promoter inserted DNA. Reactions were run for 12 hours. Both MEGAscript T7 and AmpliScribe T7 were used to drive transcription. Reactions were incubated with DNAse for 1 hour subsequent to transcription. RNA was quantified using a Qubit High Sensitivity RNA Assay kit. RNA analysis was performed using a High Sensitivity Pico mRNA Bioanalyzer.

Size exclusion included sub-17 nt size exclusion; sub-200 nt size exclusion' incubation for 10 minutes at 65°C followed by sub-17 nt size exclusion; and unexcluded control. Products were run on formaldehyde (denaturing) agarose gel.

Results are depicted in TABLE 1, below.

| Table 1 | | |
|---|---|---|
| **Sample** | **RNA (ng)** | **% yield** |
| MEGAscript >17 nt | 716 | 85 |
| Ampliscribe >17 nt | 714 | 78 |
| MEGAscript >200 nt | 637 | 76 |
| Ampliscribe >200 nt | 658 | 72 |
| MEGAscript 65C, >17 nt | 690 | 82 |
| Ampliscribe 65C, >17 nt | 573 | 62 |
| MEGAscript control | 838 | 100 |
| Ampliscribe control | 918 | 100 |

Results are graphically presented in Figure 10.

Bead-based size selection was also accomplished. In vitro transcription was performed on 1 ng DNA samples comprising T7 promoter inserted DNA. Reactions were run for 12 hours. Both MEGAscript T7 and AmpliScribe T7 were used to drive transcription. Reactions were incubated with DNAse for 1 hour subsequent to transcription. Reactions were incubated as follows: 50uL starting volume, 2ng RNA output, with 0.5x, 0.6x, 0.8x, 1×, 2x, 3x, and control (unselected). RNA was quantified using a Qubit High Sensitivity RNA Assay kit. RNA analysis was performed using a High Sensitivity Pico mRNA Bioanalyzer.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

### ASPECTS OF THE INVENTION

1. A method of determining a nucleic acid sequence adjacent to a nucleic acid sequence of interest comprising: (a) inserting a targeting nucleic acid sequence comprising a targeting sequence and a promoter into one or more specific locations in the nucleic acid sequence of interest, (b) directing synthesis of a nucleic acid from the promoter, and (c) sequencing the synthesized nucleic acid.
2. The method of aspect 1, wherein the targeting sequence comprises at least one of a clustered regularly interspaced short palindromic repeats (CRISPR) sequence, a zinc finger nuclease (ZFN) sequence, and a transcription activator-like effector nucleases (TALENs) sequence.
3. The composition of aspect 2, wherein the CRISPR sequence comprises a guide RNA with a sequence comprising SEQ ID NO: 3.
4. The method of aspect 1, wherein the promoter comprises at least one of a bacterial promoter, a viral promoter, and a eukaryotic promoter.
5. The method of aspect 4, wherein the bacterial promoter comprises at least one of araBAD, trp, lac, and Ptac.
6. The method of aspect 4, wherein the viral promoter comprises at least one of T7, T7lac, SP6, pL, CMV, SV40, and CaMV35S.
7. The method of aspect 4, wherein eukaryotic promoter comprises at least one of EF1a, PGK1, Ubc, beta actin, CAG, TRE, UAS, Ac5, Polyhedrin, CaMKIIa, GAL1, GAL10, TEF1, GDS, ADH1, Ubi, H1, and U6.
8. The method of aspect 1, wherein the sequence of interest comprises a low-complexity nucleic acid sequence.
9. The method of aspect 1, wherein the sequence of interest comprises a repetitive nucleic acid sequence.
10. The method of aspect 1, wherein the sequence of interest comprises at least one of tri-nucleotide repeat, tandem repeat, and human leukocyte antigen gene.
11. The method of aspect 1, wherein the sequence of interest comprises a mobile genetic element.
12. The method of aspect 11, wherein the mobile genetic element comprises a transposon, a retrotransposon, a DNA transposon, an insertion sequence, a plasmid, a bacteriophage, a group II intron, a group I intron, an Alu element, a MIR element, an intracisternal A particle (IAP), an ETn, a virus, or a fragment thereof.
13. The method of aspect 12, wherein the retrotransposon comprises at least one of transposable element, a LINE, a SINE, and fragments thereof.
14. The method of aspect 13, wherein the LINE comprises SEQ ID NO: 1.
15. The method of aspect 12, wherein the virus comprises at least one of a retrovirus and fragments thereof.
16. The method of aspect 1, wherein nucleic acid synthesis comprises at least one of RNA transcription and DNA synthesis.
17. The method of aspect 16, wherein RNA transcription comprises use of a RNA polymerase.
18. The method of aspect 17, wherein the RNA polymerase comprises at least one of a T7 RNA polymerase, a T3 RNA polymerase, a SP6 RNA polymerase, a RNA polymerase I, a RNA polymerase II, a RNA polymerase III, a RNA polymerase IV, a RNA polymerase V, and a single subunit RNA polymerase.
19. The method of aspect 1, wherein DNA synthesis comprises use of a DNA polymerase.
20. The method of aspect 19, wherein the DNA polymerase comprises at least one of a T7 DNA polymerase, a T3 DNA polymerase, a SP6 DNA polymerase, a DNA polymerase I, a DNA polymerase II, a DNA polymerase III, a Taq DNA polymerase, and a Pfu DNA polymerase.
21. The method of aspect 1, wherein the nucleic acid synthesis requires a primer.
22. The method aspect 1, wherein the synthesized nucleic acid is synthesized directly from the nucleic acid sequence of interest.
23. The method of aspect 1, wherein the nucleic acid is synthesized without introducing a mutation.
24. The method of aspect 23, wherein the mutation is at least one of a point mutation, a deletion, an insertion, and a chimera.
25. The method of aspect 1, wherein the synthesized nucleic acid comprises DNA.
26. The method of aspect 1, wherein the synthesized nucleic acid comprises cDNA.
27. The method of aspect 25 or aspect 26, wherein the synthesized nucleic acid is treated with an RNase.
28. The method of aspect 1, wherein the synthesized nucleic acid is a RNA.
29. The method of aspect 28, wherein the synthesized nucleic acid is treated with a DNase.
30. The method of aspect 1, wherein the sequencing comprises at least one of Sanger sequencing, Next-generation sequencing, pyrosequencing, Massively parallel signature sequencing, single molecule real-time sequencing, ion torrent sequencing, sequencing by synthesis, and sequencing by ligation.
31. The method of aspect 1, wherein the method detects a mutation in a subject.
32. The method of aspect 1, wherein the method detects a mutation in a tissue sample obtained from a subject.
33. The method of aspect 32, wherein the tissue sample comprises at least one of tumor, blood, saliva, sputum, skin, and epithelial tissue.

## Claims

1. A composition comprising (i) a first nucleic acid having a targeting sequence and a promoter, and (ii) a second nucleic acid comprising one or more repeat elements, wherein the targeting sequence comprises at least one of clustered regularly interspaced short palindromic repeats (CRISPR) sequence, a zinc finger nuclease (ZFN) sequence, and a transcription activator-like effector nucleases (TALENs) sequence that directs insertion of the first nucleic into one or more repeat elements of the second nucleic acid, and wherein the promoter comprises a nucleic acid sequence that directs RNA transcription of sequence adjacent to the one or more repeat elements of the second nucleic acid.

2. The composition of claim 1, wherein the promoter comprises at least one of a bacterial promoter, a viral promoter, and a eukaryotic promoter.

3. The composition of claim 2, wherein the bacterial promoter comprises at least one of araBAD, trp, lac, and Ptac.

4. The composition of claim 2, wherein the viral promoter comprises at least one or T7, T7lac, SP6, pL, CMV, SV40, and CaMV35S.

5. The composition of claim 2, wherein the eukaryotic promoter comprises at least one of EF1a, PGK1, Ubc, beta actin, CAG, TRE, UAS, Ac5, Polyhedrin, CaMKIIa, GAL1, GAL10, TEF1, GDS, ADH1, Ubi, H1, and U6.

6. The composition of claim 1, wherein the one or more repeat elements comprise a low-complexity nucleic acid sequence.

7. The composition of claim 1, wherein the one or more repeat elements comprise a repetitive nucleic acid sequence.

8. The composition of claim 1, wherein the one or more repeat elements comprise at least one of a tri-nucleotide repeat, a tandem repeat, and a human leukocyte antigen gene.

9. The composition of claim 1, wherein the one or more repeat elements comprise a mobile genetic element.

10. The composition of claim 9 wherein the mobile genetic element comprises a transposon, a retrotransposon, a DNA transposon, an insertion sequence, a plasmid, a bacteriophage, a group II intron, a group I intron, an Alu element, a MIR element, an intracisternal A particle (IAP), an ETn, a virus, or a fragment thereof.

11. The composition of claim 1 further comprising a RNA polymerase.

12. The composition of claim 11, wherein the RNA polymerase comprises at least one of a T7 RNA polymerase, a T3 RNA polymerase, a SP6 RNA polymerase, a RNA polymerase I, a RNA polymerase II, a RNA polymerase III, a RNA polymerase IV, a RNA polymerase V, and a single subunit RNA polymerase.

13. The composition of claim 1, further comprising reagents needed for nucleic acid sequencing.

14. The composition of claim 13, wherein the sequencing comprises at least one or Sanger sequencing, Next-generation sequencing, pyrosequencing, Massively parallel signature sequencing, single molecule real-time sequencing, ion torrent sequencing, sequencing by synthesis, and sequencing by ligation.
